# EUROPEAN PATENT SPECIFICATION

(11) **EP 4 590 222 B1**
(45) Date of publication and mention of the grant of the patent: **03.06.2026**
(21) Application number: 23768808.0
(22) Date of filing: 06.09.2023
(51) Int. Cl.: A61B 18/14, A61B 18/18, A61B 18/00, A61B 18/12

(54) **ELECTROSURGICAL INSTRUMENT AND ELECTROSURGICAL APPARATUS**
ELEKTROCHIRURGISCHES INSTRUMENT UND ELEKTROCHIRURGISCHE VORRICHTUNG
INSTRUMENT ÉLECTROCHIRURGICAL ET APPAREIL ÉLECTROCHIRURGICAL

(30) Priority: 23.09.2022 GB 202213954
(43) Date of publication of application: 30.07.2025
(73) Proprietor: Creo Medical Limited, Chepstow, Wales NP16 5UH (GB)
(72) Inventor: ULLRICH, George Christian, Chepstow, NP16 5UH (GB); THOMAS, Steven, Chepstow, NP16 5UH (GB); TURNER, Louis, Chepstow, NP16 5UH (GB); JONES, Warren, Chepstow, NP16 5UH (GB); HANCOCK, Christopher Paul, Chepstow, NP16 5UH (GB); FITZSIMONS, Duncan James Foster, St. Albans, Hertfordshire AL1 1LJ (GB)
(74) Representative: Mewburn Ellis LLP
(86) International application number: PCT/EP2023/074491
(87) International publication number: WO 2024/061631

(56) References cited:
- EP-A1- 3 988 036
- US-A1- 2013 144 284
- US-A1- 2018 280 084
- US-A1- 2019 201 095
- US-A1- 2020 253 664

## Description

### FIELD OF THE INVENTION

The invention relates to an electrosurgical instrument for sealing and cutting tissue. The electrosurgical instrument is configured to grasp biological tissue and deliver microwave energy into the grasped tissue to seal the tissue by coagulation or cauterisation. The electrosurgical instrument may be used to apply pressure to close one or more blood vessels before applying electromagnetic radiation (preferably microwave energy) to seal the blood vessel(s). The electrosurgical instrument may also be arranged to cut, e.g. separate or divide, the vessel or surrounding tissue after coagulation or sealing, e.g. using radiofrequency (RF) energy and/or a mechanical cutting element, such as a blade. The invention may be applied to a vessel sealer for use in laparoscopic surgery or open surgery as well as to an endoscopic instrument.

The invention also relates to an electrosurgical apparatus for sealing and cutting tissue which comprises a generator unit for generating radiofrequency and/or microwave electromagnetic energy, and the electrosurgical instrument.

### BACKGROUND TO THE INVENTION

Electrosurgical instruments for delivering heat energy into grasped biological tissue are known. For example, it is known to deliver microwave energy from a bipolar electrode arrangement in the jaws of a forceps. The microwave energy may be used to seal a vessel by thermal denaturation of extracellular matrix proteins (e.g. collagen) within the vessel wall. The heat energy may also cauterise the grasped tissue and facilitate coagulation.

Such devices typically find application on the end of minimally invasive surgical laparoscopic tools but can equally find use in other clinical procedural areas such as gynaecology, endourology, gastrointestinal surgery, ENT procedures, or endoscopic procedures. Depending on the context of use, these devices can have differing physical construction, size, scale and complexity.

For example, a gastrointestinal instrument might be nominally of 3 mm diameter mounted on to the end of a very long flexible shaft. In contrast, a laparoscopic instrument may be used on the end of an industry standard nominal 5mm or 10mm diameter rigid or steerable steel shaft.

US 6,585,735 describes an endoscopic bipolar forceps in which the jaws of the forceps are arranged to conduct bipolar energy through the tissue held therebetween.

EP 2 233 098 describes microwave forceps for sealing tissue in which the sealing surfaces of the jaws include one or more microwave antennas for radiating microwave energy into tissue grasped between the jaws of the forceps.

WO 2015/097472 describes electrosurgical forceps in which one or more pairs of non-resonant unbalanced lossy transmission line structures are arranged on the inner surface of a pair of jaws.

US 2018/280084 A1 describes an electrosurgical vessel sealing device that can seal biological vessels using a confined microwave field that yields a well-defined seal location with low thermal margin.

EP 3 988 036 A1 describes an object to provide a cutting device, forceps, a surgical system, a medical system, a robot, a surgical medical robot, and a surgical system capable of contacting (gripping) a target portion by a series of operations and cutting the target portion.

US 2013/144284 A1 describes an end effector assembly for microwave forceps. The end effector assembly includes a pair of opposing jaw members movable from a first position in spaced relation relative to one another to at least one subsequent position wherein the jaw members cooperate to grasp tissue therebetween.

US 2020/253664 A1 describes an electrosurgical apparatus comprising an electrosurgical forceps instrument that combines a robust jaw opening mechanism with a microwave energy delivery mechanism.

US 2019/201095 A1 describes an electrosurgical instrument with a radiating tip portion having a relative permeability and/or relative permittivity that is selected to provide an electrical length for the radiating tip portion that enables effective delivery into biological tissue of microwave EM energy supplied thereto, at two or more frequencies of choice.

### SUMMARY OF THE INVENTION

The invention provides electrosurgical instruments according to claims 1 and 7. Preferred embodiments are defined in the dependent claims.

### SUMMARY OF THE DISCLOSURE

At its most general, the present disclosure provides an electrosurgical instrument that can seal biological tissue, such as (blood) vessels, using a confined microwave field that can yield a well-defined seal location with low thermal margin. Moreover, the electrosurgical instruments may provide additional functionality, such as by a cutting device to enable fine tissue cutting and dissection to be performed. With these additional functions, fewer device interchanges may be needed during a procedure.

The electrosurgical instruments disclosed herein may be used in any type of surgical procedure, but it is expected to find particular utility for non-invasive or minimally invasive procedures. For example, the device may be configured to be introduced to a treatment site through an instrument channel of a surgical scoping device, such as a laparoscope or an endoscope.

According to a first aspect of the present disclosure, there is provided an electrosurgical instrument for sealing and cutting tissue which comprises an instrument shaft, a first jaw, a second jaw, a first electrode, a second electrode, a first isolating portion, and a cutting device. The instrument shaft comprises a coaxial transmission line for conveying microwave electromagnetic energy. The first jaw is attached to the instrument shaft and includes a first surface. The second jaw is attached to the instrument shaft and includes a second surface. The first isolating portion electrically isolates the first electrode from the second electrode. The cutting devise is configured to cut tissue. The first jaw and the second jaw can be moved between an open position, in which the tissue can be inserted between the first surface and the second surface, and a closed position, in which the first and second surfaces are brought together to clamp, hold and/or grasp tissue therebetween. The first electrode and the second electrode are arranged on the first jaw. Sections of the first and second electrodes are exposed on the first surface to define a first sealing area and a second sealing area on the first surface. The first sealing area is spaced from the second sealing area to form a gap therebetween on the first surface so that the tissue between the first sealing area and the second sealing area (e.g. some or all of the tissue in the gap) is not sealed upon the emission of the microwave electromagnetic radiation. The first sealing area and the second sealing area are configured to seal tissue on either side of the gap using the emitted microwave electromagnetic radiation. The cutting devise is effective along a cutting line on the first surface to cut the tissue in the closed position, wherein the cutting line is positioned on the gap.

The first and second electrodes in the pair of jaws operate to provide two localised seals for a biological vessel/tissue gripped between the first and second jaws, and the cutting device is operable to cut and/or divide the vessel between the two localised seals.

In use, the electrosurgical instrument may thus perform vessel/tissue sealing and vessel/tissue dividing. Vessel/tissue sealing is typically the application of pressure to squash the walls of a biological vessel together, followed by the application of some form of thermal energy. The thermal energy is applied by the first and second electrodes to the gripped tissue using the microwave electromagnetic energy. The pressure to the tissue can be applied by the first and/or second electrodes (e.g. the first and second sealing areas) and/or other parts of the first and second jaws. The applied electromagnetic energy disrupts/denatures the tissue cells and forms an amalgam of collagen predominant in vessel/tissue walls, which effectively bonds the vessel/tissue walls together. With time, post operatively, cellular recovery and regrowth occurs to reinforce the seal further. Vessel/tissue dividing is a process of cutting through a continuous biological vessel/tissue to separate it into two pieces. It is normally performed after a vessel/tissue is first sealed. Vessel/tissue dividing is performed by the cutting device, which is discussed in more detail below. As described above, the vessel/tissue dividing is locally offset from the vessel/tissue sealing. In particular, the vessel/tissue dividing occurs between the vessel/tissue seals. Stated differently, the instrument is configured such that vessel/tissue dividing occurs at a different location to vessel/tissue sealing.

Herein, the terms "proximal" and "distal" refer to the ends of the electrosurgical instrument, the shaft, and/or the coaxial transmission line further from and closer to a treatment site respectively. Thus, in use the proximal end is closer to a generator unit for providing the RF and/or microwave energy, whereas the distal end is closer to the treatment site, i.e. the patient.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction. The term "inner" means radially closer to the centre (e.g. axis) of the instrument channel. The term "outer" means radially further from the centre (axis) of the instrument channel.

The term "electrosurgical" is used in relation an instrument, apparatus or tool which is used during surgery and which utilises radiofrequency (RF) electromagnetic (EM) energy and/or microwave EM energy. Herein, RF EM energy may mean a stable fixed frequency in a range 10 kHz to 300 MHz, preferably in a range from 100 kHz to 5MHz, and more preferably in a range from 360 to 440 kHz. The microwave EM energy may mean electromagnetic energy having a stable fixed frequency in the range 300 MHz to 100 GHz. The RF EM energy should have a frequency high enough to prevent the energy from causing nerve stimulation. In use, the magnitude of the RF EM energy and the duration for which it is applied may be selected to prevent the energy from causing tissue blanching or unnecessary thermal margin or damage to the tissue structure. Preferred spot frequencies for the RF EM energy include any one or more of: 100 kHz, 250 kHz, 400 kHz, 500 kHz, 1 MHz, 5 MHz. Preferred spot frequencies for the microwave EM energy include 915 MHz, 2.45 GHz, 5.8 GHz, 14.5 GHz, 24 GHz. 2.45 GHz and/or 5.8 GHz may be preferred.

The microwave electromagnetic energy and radiofrequency electromagnetic energy may be conveyed along a common signal pathway through the instrument shaft. For example, a coaxial cable may provide the common signal pathway for conveying both the microwave energy and the radiofrequency energy. In this arrangement, the transmission line may comprise an inductive filter for blocking the microwave energy from the cutting element, and a capacitive filter for blocking the radiofrequency energy from the first and second electrodes. In an alternative arrangement, the radiofrequency energy and microwave energy are conveyed along separate pathways within the instrument shaft (the transmission line includes separate pathways), wherein the inductive filter and capacitive filter are provided at a proximal end of the instrument shaft, e.g. in a handle. For example, a coaxial cable is provided for conveying the microwave electromagnetic energy while two or more wires are provided for conveying the radiofrequency electromagnetic energy.

The instrument shaft may be dimensioned to fit within an instrument channel of a surgical scoping device. The surgical scoping device may be a laparoscope or an endoscope. Surgical scoping devices are typically provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube). The instrument channel may have a diameter suitable for receiving invasive surgical tools. The diameter of the instrument channel may be equal to or less than 13 mm, preferably equal to or less than 10 mm, and more preferably, especially for flexible insertion tubes, equal to or less than 5 mm.

The instrument shaft and the transmission line may be flexible so that they can be inserted into the instrument channel of the scoping device. Further, the transmission line may be arranged within a lumen of the shaft. The instrument shaft may cover and/or shield the transmission line. The transmission line may extend from a distal end to a proximal end of the electrosurgical instrument. In particular, the transmission line electrically connects the first electrode and the second electrode to the generator unit.

The electrosurgical instrument discussed herein may find applicability in other tissue welding techniques. For example, the energy delivery structure may be used as an alternative to staples. In some abdominal procedures, staple guns are used to deliver 50 to 100 small staples that are fired simultaneously between jaws that can have a length of 70 mm or more, or from an annular jawed arrangements with diameters of 20 to 50 mm. In this type of application multiple antenna structures such as those discussed herein may be used to cover the required length. The antenna structures may be arranged in any number of array forms to be activated simultaneously, sequentially or progressively in a suitable manner.

The first jaw and/or the second jaw may be movable relative to their instrument shaft. The first jaw and/or the second jaw may be attached to the instrument shaft via a joint or hinge. The joint may include a pivot axis around which the first jaw and/or the second jaw may rotate. The first jaw and/or the second jaw may be activated by one or more actuation rods or control wires respectively connected to the first jaw and/or the second jaw. The one or more actuation rods or control wires may extend within the instrument shaft to a proximal end of the electrosurgical instrument. The one or more actuation rods may be connected to a handle with which the first and/or second jaws can be actuated, e.g. opened and/or closed. The electrosurgical instrument comprises an actuation mechanism which converts a back-and-forth movement of the actuation rod(s) or control wire(s) in a rotational movement of the first jaw and/or the second jaw.

For example, both jaws can be movable, e.g. rotatable around a (common) pivot axle. In another embodiment, one of the jaws is fixed to the shaft and the other jaw is movable relative to the one jaw.

In the open position, the first jaw and the second jaw are (maximally) spaced apart so that there is a free space between the first surface of the first jaw and the second surface of the second jaw. In this way, tissue can be inserted between the first surface and the second surface in the open position. Usually, the first jaw and the second jaw are moved towards the tissue such that the tissue is pushed into the space between the first surface and the second surface in the open position of the first jaw and the second jaw.

By moving the first jaw and/or the second jaw from the open position to the closed position, the tissue between the first surface and the second surface can be grasped and/or clamped between the first surface and the second surface. In this way, the tissue can be fixed between the first surface and the second surface in the closed position. The first surface and the second surface are the faces of the first jaw and the second jaw, respectively, that face each other in the open and/or closed position.

The pair of jaws may be pivotable relative to each other about a hinge axis that lies transverse to a longitudinal axis of the coaxial transmission line. In one example, the pair of jaws comprises a static jaw that is fixed relative to the instrument shaft, and a movable jaw that is pivotably mounted relative to the static jaw to open and close the gap between the opposing inner surfaces. The energy delivery structure may be disposed on the inner surface of the static jaw. In another example, both jaws are arranged to pivot with respect to the instrument shaft, e.g. in a symmetrical forceps-type or scissors-type arrangement. Relative movement of the pair of jaws may be controlled from a handle at a proximal end of the instrument shaft. A control rod or control wires may pass through the instrument shaft to operably couple an actuation mechanism on the handle to the pair of jaws.

In another example, the pair of jaws may be arranged to move relative to one another in a manner that maintains the inner surfaces thereof in an aligned, e.g. parallel, orientation. This configuration may be desirable for maintaining a uniform pressure on grasped tissue along the length of the jaws. One example of such a closure mechanism is disclosed in WO 2015/097472.

The first jaw and/or the second jaw may have a Maryland configuration. This can include that the first jaw and the second jaw are not straight but bent/curved, e.g. forming an arc or an S-shape in a side view.

The first surface includes the exposed sections of the first electrode and the second electrode. The first electrode and/or the second electrode are arranged within and/or on the first jaw. The first electrode and/or the second electrode are made from an electrically conductive material, such as metal, and may be connected to an inner conductor and an outer conductor of the coaxial cable, respectively.

The first electrode and the second electrode are electrically isolated from each other by the first isolating portion. Further, the sections of first electrode and the second electrode that are exposed on the first surface are spaced apart from each other, for example by an air gap or an exposed section of the first isolating portion.

The exposed sections of the first electrode and/or the second electrode may extend as lines and/or form areas on the first surface. In particular, the exposed sections of the first electrode and the second electrode define two sealing areas laterally offset on the first surface, namely the first sealing area and the second sealing area. The first sealing area and the second sealing area may be connected to each other on the first surface by the other parts of the exposed sections of the first electrode and the second electrode. This means that the first sealing area and the second sealing area may or may not be connected to each other on the first surface.

In any case, the first sealing area and the second sealing area are separated by a gap on the first surface. For example, the first sealing area includes a first set of exposed sections of the first electrode and the second electrode which extends along side (e.g. and spaced from, in a side-by-side manner) the second sealing area, wherein the second sealing area includes a second set of exposed sections of the first electrode and the second electrode. For example, the first sealing area and/or the second sealing area define elongated structures (e.g. rectangles or ellipses) that are arranged side-by-side and separated by the gap on the first surface.

The exposed sections of the first electrode may form (straight) lines which are separated by the gap. The exposed sections of the second electrode may form (straight) lines between which the exposed sections of the first electrode are positioned. Exposed sections of the first isolating portion may be arranged between the (straight) lines of exposed sections of the first electrode and the second electrode.

The first sealing area and the second sealing area are each configured to emit microwave energy at the exposed sections of the first electrode and the second electrode. For example, the exposed section of the first electrode may be considered an active electrode and the exposed section of the second electrode may be a dipole antenna for radiating microwave electromagnetic energy. This applies for both the first sealing area and the second sealing area. This means that both the first sealing area and the second sealing area include two electrodes of different polarity or electrical potential.

The first sealing area and the second sealing area are configured to emit microwave energy to tissue that is close to or in contact with the exposed sections of the first electrode and the second electrode. The exposed sections of the first electrode and the second electrode are arranged such that tissue that is clamped and/or grasped between first surface and the second surface in the closed position is in contact with the exposed sections of the first electrode and the second electrode or in contact with the first sealing area and the second sealing area. This means that the first sealing area and the second sealing area have a double functionality. They can clamp or grasp tissue (with the second surface being the counterpart) and emit microwave energy.

The greatest intensity of the emitted microwave energy is achieved in a portion of the tissue that is in contact with or directly above the exposed sections of the first electrode and the second electrode in the first sealing area and the second sealing area. In particular, the intensity of the emitted microwave energy is highest at respective edges or corners of the exposed sections of the first electrode and the second electrode that face each other. In other words, the intensity of the emitted microwave energy is highest above the interface of the first isolating portion and the first electrode (at the first surface) and at the interface of the first isolating portion and the second electrode (at the first surface). Thus, portions of the exposed sections of the first electrode that are away from the respective exposed sections of the second electrode exhibit a (significantly) lower intensity of the emitted microwave energy. These portions may be regarded as not corresponding to the first and/or second sealing areas since, due to the reduced intensity, no or insufficient microwave sealing can be achieved.

Further, (significantly) less microwave energy is absorbed by tissue that is arranged above or in contact with the area of the first surface that is arranged between the first sealing surface and the second sealing surface, i.e. in the area of the gap. This may result in that tissue is only sealed above the first sealing area and the second sealing area but tissue in an area between the first sealing area and the second sealing area is not sealed. In other words, if an intensity of the electromagnetic energy emitted directly at the exposed sections of the first electrode and the second electrode (i.e. at the first sealing area and the second sealing area) is set to 100%, the intensity of the electric energy measured in or above the gap (i.e. an area between the first sealing area and the second sealing area) is 60%, 50%, 40%, 30%, 25%, 20%, 10%, 5%, or less.

The intensity of the emitted microwave radiation is only sufficiently high to effect tissue sealing above the first sealing area and the second sealing area whereas the intensity of the emitted microwave energy is below a threshold for effecting tissue sealing in an area between the first sealing area and the second sealing area. The threshold for effecting tissue sealing depends on the tissue. However, due to the separation of the first sealing area and the second sealing area, an area between the first sealing area and the second sealing area can be established in which no or little (e.g. only negligible) tissue sealing is effected upon the emission of microwave energy. In other words, a dimension of the gap is chosen such that effective tissue sealing can be achieved over the first sealing area the second sealing area but not in an area therebetween.

The cutting device is configured to cut or divide tissue that is arranged between the first sealing area and the second sealing area. The line of the cut or divide of the tissue may be considered the cutting line. The cutting device may be arranged on or can be moved along the cutting line. The cutting line is arranged between the first sealing area and the second sealing area, i.e. within the gap. Optionally, the cutting line is arranged in the middle between the first sealing area and the second sealing area so that a distance from the cutting line to the first sealing area is the same as a distance from the cutting line to the second sealing area.

The cutting device is therefore configured to cut tissue that is not sealed. For example, the tissue is firstly sealed and then cut. Due to the arrangement of the cutting device in the gap, the cutting device cuts in a portion of the tissue that is not sealed although other portions of the tissue have been sealed due to the emission of microwave energy. This allows that sealing and cutting of the tissue may be simultaneously conducted because little or no interaction between the sealing at the cutting is expected due to the spatial separation of the sealing at the cutting.

In an optional embodiment, the second electrode covers the first electrode on the side of the first electrode facing away from the first surface and/or to first isolating portion is arranged between the first electrode and the second electrode.

The second electrode may completely cover the first electrode on one side of the first surface so that the second electrode shields the first electrode. The second electrode may act and/or form a half-shell or channel in which the first electrode is arranged. For example, the second electrode may be a ground electrode. In the closed position, the second electrode is configured to shield microwave radiation that is emitted from the first electrode away from the first surface. Thus, the first electrode may solely be configured to emit microwave energy through or on the first surface.

In an optional embodiment, portions of the first electrode and/or the second electrode are plate-shaped and respectively include end faces, wherein optionally the end faces form the exposed sections. Optionally, in a cross-sectional view of the first jaw, the portions of the first electrode and/or the second electrode are U-shaped or V-shaped.

For example, the portions of the first electrode and/or the second electrode that extend along sections of the first jaw where the first and second sealing areas are provided may be plate-shaped. Other portions of the first electrode and/or the second electrode may have different configurations. For example, proximal and/or distal end portions of the first electrode and/or the second electrode may have shapes that deviate from a plate shape. This may be provided for forming distal and/or proximal end portions of the jaw. End faces of the plate-shaped portions of the first electrode and/or the second electrode can be in the exposed sections on the first surface.

The plate-shaped portions of the first electrode and/or the second electrode may have a shape of the letter U, V, or variations thereof in a cross-sectional view of the first jaw. For example, the first electrode may be arranged within the shape defined by the U-shape or V-shape in a cross-sectional view.

In an optional embodiment, the exposed sections of the first electrode and the second electrode at least partially extend parallel to each other, each include two straight portions that extends parallel to each other, and/or each form a loop.

The exposed sections of the first electrode and the second electrode completely or partially extend parallel to each other on the first surface. For example, two portions of the exposed sections are straight which are connected by a connecting section - thus forming a loop. Other shapes of the loops are possible. The exposed sections of the first electrode may completely or partially extend parallel to the exposed section of the second electrode. The straight portions of the exposed sections may form the first sealing area and the second sealing area. So, the distance between straight portions of the exposed sections defines the width of the gap. In case of a loop, the first electrode and/or the second electrode each form a single section that is exposed on the first surface.

In an optional embodiment, the first electrode and the second electrode are exposed on the first surface and on the distal end face, wherein optionally, in the closed position, the second jaw covers the exposed first and/or second electrodes on the first surface and the distal end face.

The first and second electrodes in the pair of jaws can operate to provide one or more (e.g. two) localised seals for a biological vessel/tissue gripped between the first and second jaws. Further, the first electrode and the second electrode can be used for radiofrequency cutting at the distal end face. In the open position of the first and second jaws in which the second jaw does not cover the section of the first electrode and the second electrode exposed on the distal end face, the sections of the first electrode and the second electrode that are exposed on the distal end face can act as an active electrode and a return electrode for radiofrequency cutting. This may allow fine cutting at the distal end face of the electrosurgical instrument, for example, for cutting a hole into tissue so that the electrosurgical instrument can be further advanced into and/or through the tissue, for example, as part of a tunnelling procedure. Further, the first and second electrodes exposed on the distal end face can be used to cut fine or small sections of tissue that are clamped or grasped between the distal end face and the second jaw. Thus, tissue can be "nibbled".

In addition to the above-described modes of use, the disclosure may allow for another possible mode of cutting tissue by the mechanically moving a centre cutter (e.g. a blade) instead of radiofrequency energy. The geometry of an antenna (e.g. the first and second electrode) of the first jaw in this configuration may allow a mechanically moving blade (or moving radiofrequency electrode) in the centre channel or groove to move straight forward right up to, or possibly even a little beyond a distal end face of the first jaw, whilst still safely covered over by the second jaw at the distal end face (e.g. an overhang portion). This action might allow, for example, simultaneous radiofrequency cutting or microwave sealing (from the antenna) and mechanical cutting (from the blade) right up to the tip (e.g. a distal end face) between the first jaw and the second jaw. This enables tissue to be sealed and "nibbled" without having to rely only on radiofrequency energy as the means to part tissue.

The first surface includes some exposed sections of the first electrode and the second electrode. Other sections of the first electrode and the second electrode are exposed on the distal end face. The first electrode and/or the second electrode are arranged within and/or on the first jaw. The first electrode and/or the second electrode are made from an electrically conductive material, such as metal, and may be connected to an inner conductor and an outer conductor of the coaxial cable, respectively.

The first electrode and the second electrode are electrically isolated from each other by the first isolating portion. Further, the sections of the first electrode and the second electrode that are exposed on the first surface and/or the distal end face are spaced apart from each other, for example by an air gap or the first isolating portion (e.g. an exposed section thereof).

The first surface and the distal end face may form a continuous surface of the first jaw. The first surface and the distal end face may be inclined relative to each other. For example, an angle between a plane defined by the first surface and a plane defined by the distal end face may form an angle between 10° to 90°, optionally 45°, 60°, or 90°. The distal end face, the first surface, and an outer surface of the second electrode may define an outer surface of the first jaw. In this configuration, the distal end face is a side surface while the first surface and the outer surface of the second electrode are surfaces that extend along the longitudinal direction of the first jaw. In other words, the distal end face extends transverse to the longitudinal direction of the first jaw. The distal end face may be a surface of the first jaw that is arranged at a distalmost position of the first jaw. In other words, when moving the first jaw along the longitudinal direction of the first jaw towards the tissue, the distal end face firstly and/or solely contacts the tissue.

The distal end face may be straight/flat. Alternatively, the distal end face may be curved. The sections of the first electrode, the second electrode, and the first isolating portion that are exposed at the distal end face may be flush with respect to each other or define a flat surface. Alternatively, the sections of the first electrode and/or the second electrode that are exposed at the distal end face may protrude from the section of the second isolating portion that is exposed at the distal end face.

The second jaw may partially or completely cover the first surface and a distal end face in the closed position of the first jaw and the second jaw. In any case, the second jaw covers the section of the first electrode that is exposed at the distal end face and/or the section of the second electrode that is exposed to the distal end face in a closed position. Stated differently, if the first jaw and the second jaw are brought together with no tissue therebetween, the second jaw covers and/or contacts the sections of the first electrode and/or the second electrode that are exposed on the first surface and/or the distal end face. In this way, the second jaw may function to shield the first and/or second electrode(s) when the jaws are closed, for example, to avoid unintentionally treating tissue whilst the instrument is moved into position at a treatment site.

The sections of the first electrode and the second electrode exposed at a distal end face can define an active electrode and a return electrode, respectively, which can be used for cutting tissue at the distal end face. The first electrode and the second electrode can be connected to the transmission line which is configured to convey both microwave and radiofrequency energy.

In an optional embodiment, the second jaw includes an overhang portion protruding from the second surface. Optionally the overhang portion covers sections of the first electrode and/or the second electrode that are exposed on the distal end face in the closed position of the first jaw and the second jaw.

The overhang portion may include a side surface which is continuous with the second surface. The second surface and the side surface may be inclined to each other. For example, an angle between a plane defined by the second surface and a plane defined by the side surface may form an angle between 10° to 90°, optionally 45°, 60°, or 90°. This angle may correspond to (e.g. be identical to) the angle defined by the first surface and the distal end face. In the closed position, the distal end face and side surface may extend parallel to each other and/or contact each other.

The overhang portion of the second jaw may provide the first and second jaws with a shape similar to a hooked-shaped beak of a bird of prey. In the longitudinal direction of the first jaw and the second jaw (e.g. measured from the pivot axle), the second jaw may have a greater length along the longitudinal direction compared to the first jaw in the closed position. The difference in length between the first jaw and the second jaw can correspond to the length of the overhang portion along the longitudinal direction. In other words, without the overhang portion, the first jaw and the second jaw may have the same length and/or configuration. The overhang portion protrudes from the second surface towards the first jaw and partially or completely covers the distal end face.

Tissue can be clamped or grasped between the distal end face and the side surface of the overhang portion. The surface areas of the side surface and the distal end face are significantly smaller than the surface areas of the first surface and the second surface. Therefore, smaller portions of tissue can be grasped between the distal end face and the side surface compared to clamping tissue between first surface and the second surface. This allows finer cutting of tissue that is clamped between the distal end face and the side surface.

In an optional embodiment, the cutting line is arranged between the straight portions of the exposed sections of the first electrode in the closed position. Alternatively or additionally, the cutting line is arranged within the loop of the exposed sections of the first electrode in the closed position.

The cutting line may extend parallel between the straight portions of the exposed sections of the first electrode and/or the second electrode. In this case, the cutting line is straight. Further, the cutting line extends within the loop formed by the exposed section of the first electrode. Thus, on the first surface, the exposed section of the first electrode and the exposed section of the second electrode partially surround the cutting line.

In an optional embodiment, the electrosurgical instrument further comprises a second isolating portion. Optionally the cutting device includes a radiofrequency electrode arranged on and movable along the cutting line. Alternatively, the second isolating portion electrically isolates the radiofrequency electrode from the first electrode.

The first isolating portion and/or the second isolating portion may be made from an electrically non-conductive material such as a ceramic (e.g. Zirconia) or plastic material (e.g. Polyetheretherketon (PEEK)). The first isolating portion and/or the second isolation portion may be fixed to the first third and/or the second electrode and/or vice versa. The first electrode may be sandwiched between the first isolating portion and the second isolating portion. For example, the second isolating portion may cover the side of the first electrode that faces the first surface except for the exposed sections of the first electrode. As an example, the second isolating portion is arranged in the space formed by the U-shaped or V-shaped portions of the first electrode.

The radiofrequency electrode may be an active electrode wherein one or more of the first to fourth electrodes may act as a return electrode.

The radiofrequency electrode may be arranged on the second isolating portion or within a recess in the second isolating portion. The radiofrequency electrode may be exposed at the first surface, e.g. the second isolating portion. The cutting line therefore extends over the second isolation portion. A width of the second isolating portion along the first surface may correspond to the width of the gap. The second isolating portion may delimit the first sealing area and/or the second surface area. Optionally, a section of the second isolating portion that is exposed on the first surface is arranged between the first sealing area and the second surface area. The section of the second isolating portion that is exposed on the first surface may correspond to the gap. The cutting line may be in the middle of the section of the second isolating portion that is exposed on the first surface.

It has been found that RF cutting is less effective on tissue that has previously been sealed by microwave energy. It is thought that this is due the desiccation of the tissue caused by the microwave sealing. Due to the spatial separation of the microwave sealing and that radiofrequency cutting, this can be avoided so that efficiency of the RF cutting can be increased.

In an optional embodiment, the first sealing area and the second sealing area define an angle of less than 180°.

This means that the first sealing area and the second sealing area do not define a flat area (not considering the cutting device and the exposed second isolating portion).

For example, the area of the first surface on one side of the cutting device is inclined to an area of the first surface on the other side of the cutting device. In other example, the exposed section of the second isolating portion provides a flat surface, while the first and second sealing areas define an angle of less than 180° with the flat exposed section of the second isolating portion. In this case, only the first sealing area and the second sealing area are inclined.

The exposed section of the second isolation portion may be flat and/or define an angle with the first sealing area and the second sealing area. Alternatively, the exposed section of the second isolation portion may include two surface areas, one of which forms a flat plane with the first sealing area and the other of which forms a flat plane with the second sealing area. The cutting line may be arranged at that line where the two surface areas of the exposed section of the second isolation portion meet. Further, the two surface areas of the exposed section of the second isolation portion may be symmetrical to the cutting line and/or each define an angle with the respective first and second sealing areas.

In an optional embodiment, the radiofrequency electrode includes a ridge protruding from the second isolating portion.

In this case, the radiofrequency electrode may be a bar or rod made from an electrically conductive material (e.g. metal or metal alloy) that is arranged on or embedded in a recess of the second isolating portion. The radiofrequency electrode can be permanently fixed to the second isolating portion. The radiofrequency electrode may be arranged on the second isolating portion so that tissue can contact the radiofrequency electrode, especially in the closed position.

In an optional embodiment, the cutting device includes a radiofrequency electrode exposed on the second surface. Further optionally, the electrosurgical instrument comprises the second isolating portion arranged on the first jaw so that, in a closed position, the second isolating portion electrically isolates the radiofrequency electrode from the first electrode, wherein optionally the second isolating portion is made from silicon.

In this case, the radiofrequency electrode is arranged on the second jaw. For example, the radiofrequency electrode may be configured to be in contact or close to the second isolating portion in the closed position. The radiofrequency electrode may be electrically isolated from other parts of the second jaw, for example by a third isolating portion. The arrangement of the radiofrequency electrode on the third isolating portion may include the same configuration, features, and/or characteristics as the arrangement of the radiofrequency electrode on the second isolating portion described above.

The silicon is more flexible and softer compared to other types of electrically isolating material. This may reduce the pressure applied to the tissue between the radiofrequency electrode and the second isolating portion in the closed position. It has been found that RF cutting is more effective if less pressure is applied during microwave sealing. The reduction of pressure may be achieved by the provision of the second isolating portion made from silicon.

In an optional embodiment, the second jaw includes a first arm movable with respect to the second surface and/or the second jaw. Optionally, the second jaw and/or the second surface include an opening through which the first arm can be moved.

The first arm may also be attached to the joint. For example, the first arm may be pivotable around the pivot axis. The first arm may be moved independently from the second jaw. In an alternative embodiment, the first arm may be pivotable with respect to the second jaw, i.e. is pivotally attached to the second jaw. In this arrangement, movement of the second jaw also moves the first arm so that the first arm can be moved relative to the second jaw.

The opening of the second jaw may completely extend through the second jaw so that the first arm can be moved from one side of the second jaw, through the opening, and to the other side of the second jaw. The opening may be a through-hole which extends from the second surface to the other side of the second jaw.

The outer shape of the first arm may match the shape of the opening. There may be a small gap between the outer perimeter of the first arm and the inner surface of the opening. Alternatively, the inner surface of the opening provides a sliding contact with the first arm.

The first arm may be brought into contact with the second isolating portion or the radiofrequency electrode arranged on the first jaw in the closed position. The first arm may be configured to be not brought in contact with the first sealing area and a second sealing area (either in the closed position or the open position of the jaws). Rather, the second surface of the second jaw (i.e. not the first arm) are in contact or close to the first sealing area and the second sealing area in the closed position. The first jaw and the second jaw in the closed position provide pressure on the tissue over the area of the first sealing area and the second sealing area while little or no pressure is applied over the gap if the first arm is arranged away from the first surface in the closed position. This means that the first arm can be brought into contact with the gap arranged between the first sealing area the second sealing area.

A part of the first arm may be made of an electrically conductive material that forms a half-shell with an electrically conductive material of the second jaw (e.g. a fourth electrode) when the first arm is arranged within the opening. The electrically conductive material of the second jaw may provide a half-shell except for the opening. If the first arm is arranged within the opening, the electrically conductive materials of the first arm and of the second jaw may provide the half-shell whose shape may be symmetrical to the second electrode of the first jaw.

A fourth isolating portion may be arranged within the half-shell of the second jaw. The fourth isolating portion may contact the first electrode in the closed position and/or provides electrical isolation to the first arm. For example, the fourth isolating portion may surround the opening.

In an optional embodiment, the radiofrequency electrode is arranged on the first arm.

In this case, the radiofrequency electrode may be a ridge or bar made from an electrically conductive material that can be exposed on an isolating portion such as the third isolation portion described below. In this case, tissue can be sealed using microwave energy in the closed position of the jaws whereby the first arm can be positioned away from the first surface - for example the first arm is arranged outside of the opening. In this case, little or no pressure is applied on tissue over the area of the gap (i.e. between the first sealing area the second sealing area). For cutting the tissue, the first arm is then moved through the opening so that the radiofrequency electrode presses the tissue against the first surface, optionally the gap (i.e. in an area between the first sealing area and the second sealing area). This increases the pressure for RF cutting while the tissue to be cut is not sealed by the emission of microwave energy. Further, the tissue grasped by the first jaw and the second jaw may not be moved as the first and second jaws may remain in the closed position during both microwave sealing and radiofrequency cutting. This helps to reliably cut between the sealed areas of the tissue. Additionally, differing levels of pressure may be applied during sealing and cutting. For example, a higher pressure may improve a seal, but a lower pressure may improve a cut.

In an optional embodiment, the first arm includes a third isolating portion which is in contact with the radiofrequency electrode in a press position of the first arm, in which the first arm and the first surface are brought together to cut tissue therebetween.

In this case, tissue can be sealed using microwave energy in the closed position whereby the first arm can be positioned away from the first surface - for example the first arm is arranged outside of the opening. In this case, little or no pressure is applied on tissue over the area of the gap (i.e. between the first sealing area the second sealing area) and/or over the third/radiofrequency electrode arranged on the second isolating portion. For cutting the tissue, the first arm is then moved through the opening so that the first arm presses the tissue against the radiofrequency electrode arranged on the first jaw. This can be considered the press position in which the pressure on the tissue can be increased for radiofrequency cutting. As before, differing levels of pressure may be applied during sealing and cutting. For example, a higher pressure may improve a seal, but a lower pressure may improve a cut.

The third isolating portion may define a face/outer surface of the first arm that faces the radiofrequency electrode. The third isolating portion may be made from the same electrically non-conductive material as the first isolating portion and/or the second isolating portion. The electrically conductive part of the first arm may support the third isolation portion.

In an optional embodiment, the second jaw includes a fourth isolating portion which covers the opening and is flexible across the opening so that the radiofrequency electrode is configured to deform the fourth isolating portion in the closed position or the first arm deforms the fourth isolating portion in the press position of the first arm, in which the first arm and the first surface are brought together to cut tissue therebetween, wherein optionally the fourth isolating portion is made from silicon.

In this embodiment, the fourth isolating portion may include the features of as described above but additionally covers the opening. The section of the fourth isolating portion that covers the opening may be thin and/or have the configuration of a membrane across the opening. This provides sufficient flexibility and/or softness of the fourth isolating portion over the opening so that the fourth isolating portion can be deformed. For example, the fourth isolating portion includes a membrane section providing a membrane that covers the opening. The membrane section may be unitary component with the rest of the fourth isolating portion.

The membrane section or the complete fourth isolating portion may be made from silicon or a silicone-based material which provides the elasticity of the membrane section.

The provision of the fourth isolating portion that covers the opening allows that the first arm is either completely made of electrically conductive material or that the portion of the first arm that faces the radiofrequency electrode can be made from electrically conductive material. In the press position, i.e. when the first arm presses against the radiofrequency electrode, the first arm and the radiofrequency electrode are separate by the (membrane) section of fourth isolating portion that covers the opening. The fourth isolating portion may also be deformed in the closed position of the first jaw and the second jaw in that the radiofrequency electrode pushes the membrane section /the fourth isolating portion into the opening.

In an optional embodiment, the second jaw includes a protruding portion and a recessed portion, wherein in the closed position, the protruding portion is in contact with the first surface and the recessed portion is spaced from the radiofrequency electrode. Optionally the protruding portion is flexible so that the second jaw can be further move towards the first jaw relative to the closed position so that the recessed portion can be moved closer towards the radiofrequency electrode.

The protruding portion and the recessed portion may have U-shape or V-shape in a cross-sectional view of the second jaw. Distal end faces of the protruding portion may contact the first sealing area and the second sealing area in the closed position, for example for grasping the tissue for microwave sealing.

The protruding portion and the recessed portion may form a half-shell. The protruding portion may be configured to contact the first sealing area and the second sealing area in the closed position. The second surface may be defined by protruding portion. The recessed portion may be surrounded by the protruding portion when viewing onto the second surface. The recessed portion may be offset from the second surface for example away from the first surface in the closed position. Thus, there is a gap between the first surface and the recessed portion in the closed position. The gap can be reduced by further pressing the second jaw against the first jaw (or vice versa) so that the protruding portion is bent. In this way, the pressure on the tissue for RF cutting can be increased compared to the closed position since the recessed portion presses more against the radiofrequency electrode arranged on the first jaw compared to the closed position.

The protruding portion is sufficiently flexible so that the recessed portion can be moved further towards the first surface. The protruding portion may be bent or flexes outwards.

The protruding portion may be made from a flexible plastic material which is provided with a conductive layer for providing the shielding capabilities of the second jaw. The recessed portion may be made from a rigid material, e.g. an electrically isolating material.

In an optional embodiment, the electrosurgical instrument includes a third electrode for emitting microwave energy which is exposed on the second surface and/or a fourth electrode for emitting microwave energy which it is exposed on the second surface.

The first electrode and/or the second electrode may be symmetrical to the third electrode and/or fourth electrode, respectively - both relative to the first and second surface in the closed position. More generally, the characteristics, features and preferred embodiment described in conjunction with the first and/or second electrodes may equally apply for the third and/or fourth electrodes, respectively. The third electrode may be electrically isolated from the fourth electrode by the fourth isolating portion. The third electrode may be covered by the third isolating portion.

In an optional embodiment, the first electrode is electrically connected to the third electrode and/or the second electrode is electrically connected to the fourth electrode.

For example, the first electrode and third electrode are electrically connected to an inner conductor of the coaxial cable while the second electrode and the fourth electrode are connected to the outer conductor of the coaxial cable. There may be a direct electrical connection between the first electrode and third electrode and/or between the second electrode and fourth electrode. Alternatively, the electrical connection is provided by the connection to the same conductors of the (coaxial) transmission line.

This arrangement allows the application of microwave energy to the tissue from two sides. If the first jaw and the second jaw are symmetrically configured, this allows a symmetrical application of microwave energy from the top and bottom after tissue being clamped in the closed position.

In an optional embodiment, the electrosurgical instrument further comprises a first coaxial cable, which is connected to the first electrode and the second electrode, and a second coaxial cable which is connected to the third electrode and the fourth electrode.

It has been observed that the emission distribution of the microwave energy varies along the extension of the exposed sections of the first electrode and the second electrode. This equally applies to the exposed sections of the third electrode and the fourth electrode. The emission distribution is frequency-dependent. To provide a more uniform distribution of the microwave energy into the tissue, the first and second electrodes receive microwave energy of a first frequency while the third and fourth electrodes receive microwave energy of a second frequency. Due to the difference in frequency, the emission distribution of the first and second electrodes differs from the emission distribution of the third and fourth electrodes. This may result in that a local minimum of the emitted microwave energy at the first jaw may be aligned with a local maximum of emitted microwave energy at a second jaw (in the closed position). This may be achieved in that the first coaxial cable is connected to a first generator generating a first frequency while the second coaxial cable is connected to a second generator generating the second frequency.

In an optional embodiment, a first point of contact of the first coaxial cable with the first electrode and second electrode is laterally offset along a direction of extension of the first jaw and the second jaw compared to the second point of contact of the second coaxial cable with the third electrode in the fourth electrode.

In this case, the first and second electrodes may emit microwave energy of the same frequencies as the frequency of the microwave energy emitted by the third and fourth electrodes. This may be implemented in that the first coaxial cable and the second coaxial cable may be connected to the same generator. A more even distribution of the emitted microwave energy is provided by laterally shifting the emission distributions of the first jaw and a second jaw by connecting the first coaxial cable to the first jaw at different point compared to the point where the second coaxial cable is attached to the second jaw. The emission distribution is a relative to the point where the coaxial cables are connected to the respective electrodes. So, changing the point of contact changes the emission distribution. For example, the overall emission distribution is the same however it is spatially shifted along the first surface or the second surface.

In an optional embodiment, the first isolating portion is made from a material different to the material from which the fourth isolating portion is made.

The emission characteristics of the first electrode and the second electrode as well as of the third electrode and the fourth electrode also depend on the dielectric medium arranged between the respective two electrodes. So, using different materials between the respective two electrodes the emission distributions can be made different to each other. Thus, a more even overall emission distribution can be achieved, even if the same frequency is applied to the first to fourth electrodes and the same points of contact are provided on the first and second jaws. For example, the first isolating portion is made from PEEK and the fourth isolating portion is made from Zirconia or vice versa.

In an optional embodiment, the cutting device includes a blade movable along the cutting line, and optionally a blade is in electrical contact with the first electrode.

The blade may be actuated by an actuation rod or control wire that can extend within the instrument shaft and is connected to the handle. The blade may have a cutting edge which is sufficiently sharp to mechanically cut tissue which is clamped between first jaw in the second jaw in the closed position.

In one example, the blade may be slidable in a longitudinal direction between a retracted position in which it lies proximal to the pair of jaws and an extended position in which it lies within the region between the pair of jaws. It is desirable for the blade to slide into the region between the blade when they are in the closed position. The blade may be slidable along a longitudinally extending recessed groove formed in the pair of jaws, i.e. in each of the first jaw and the second jaw, so that it can contact tissue held in the gap when the pair of jaws are closed. The groove may be arranged to act as a guide rail for the cutting blade, which may be particular useful where the pair of jaws curve towards their distal ends (e.g. having a Maryland configuration).

In another example, the blade may be mounted within one of the pair of jaws and may be slidable or otherwise movable in a lateral direction between a retracted position in which it lies beneath the inner surface of the jaw and an extended position in which it lies within the region between the pair of jaws.

The blade may comprise a rigid element with a sharp edge adapted to slice biological tissue, e.g. a scalpel-type blade or the like. This type of blade is configured to perform a "cold" cut, which may be preferred because it carries a low risk of collateral thermal damage that is associated with other cutting techniques. However, the disclosure need not be limited to a cold cut blade. In other examples, the blade may comprise any one of: a bipolar radiofrequency cutting element, an ultrasound sonotrode, and a heatable wire element.

The first electrode and/or third electrode may each include a groove in which the blade is movable. The grooves allow the movement of the blade in the closed position. The grooves define the cutting line. In this case, the blade may have the same electrical potential as the first electrode. Further, in this embodiment, cutting of the tissue is a mechanical cutting by the blade.

In this case, the first electrode and/or the third electrode are sufficiently wide so that the first sealing area and the second sealing area are offset from the groove or the cutting line. This means, that in this case, the sealing area is not provided by the complete exposed sections of the first electrode but only that part of the exposed sections of the first electrode that is close to the exposed sections of the second electrode since most microwave energy is emitted over the gap between the exposed sections of the first and second electrodes, i.e. over the exposed sections of the first isolating portions.

In an optional embodiment, the cutting device includes a blade movable along the cutting line, wherein the second isolating portion includes a groove in which the blade is movable.

In this case, the blade may form the radiofrequency electrode and is electrically isolated from the first electrode by the second isolating portion as described above. Here, the second isolating portion includes a groove along which the blade is movable. The groove defines the cutting line. The blade can be connected to radiofrequency energy so that the cutting can be radiofrequency cutting and/or a mechanical cutting by the blade.

The fourth isolating portion may also include a groove which matches the groove in the second isolating portion so that the blade can be moved in both grooves similar to the above-described grooves. However, in this embodiment, the second jaw does not include a third electrode and third isolating portion, but only the fourth electrode and the fourth isolating portion. The second jaw does not include an active electrode for emitting microwave energy.

Alternatively, the second jaw includes the third and fourth electrodes for emitting microwave energy as described above. The radiofrequency electrode (e.g. the blade) may an additional (fifth) electrode. In this case, the third isolating portion may include a groove which matches the groove in the second isolating portion so that the blade can be moved in both grooves similar to the above-described grooves.

According to a second aspect of the present disclosure, there is provided an electrosurgical apparatus for sealing and cutting tissue which comprises a generator unit for generating radiofrequency and/or microwave electromagnetic energy and the electromagnetic electrosurgical instrument as described above. The transmission line is configured to convey the radiofrequency and/or microwave electromagnetic energy from the generator unit to the first electrode, the second electrode and/or the cutting device.

The generator unit may be configured to generate electromagnetic energy of a fixed single frequency or of a plurality of fixed single frequencies. Alternatively or additionally, the generator unit may be tuneable to generate electromagnetic energy of various frequencies, for example in a continuous range of frequencies between a minimum frequency and a maximum frequency. The generator unit may be connected to a power supply which provides the energy for generating the radiofrequency electromagnetic energy and/or microwave electromagnetic energy.

The generator unit is electrically and/or electronically (directly or indirectly) connected to the transmission line. Optionally, the generator unit generates the radiofrequency energy and/or microwave energy which is conveyed by the transmission line to the first to fourth electrodes where the radiofrequency energy and/or microwave energy is radiated into the treatment zone.

In an optional embodiment, the generator unit is configured to simultaneously generate microwave electromagnetic energy of the first frequency and microwave electromagnetic energy of a second frequency.

For example, the generator unit includes a generator that is configured to simultaneously generate electromagnetic energy of two different (fixed) frequencies.

Alternatively, the generator unit includes a first generator for generating electromagnetic energy of the first frequency and a second generator for generating electromagnetic energy of the second frequency. The output of the first generator and output of the second generator can be combined using a multiplexer.

The multiplexer may be a diplexer and can combine the input from various sources into one output. For example, a multiplexer (or diplexer) is used to combine the output of first and second generators to a single output which is connected or coupled to the transmission line.

In an optional embodiment, the first coaxial cable is connected to the generator unit to receive the first frequency and the second coaxial cable is connected to the generated unit to receive the second frequency.

Here again, the generator unit may include a first generator for generating electromagnetic energy of the first frequency and a second generator for generating electromagnetic energy of the second frequency. The first coaxial cable is connected to the first generator and the second coaxial cable is connected to the second generator.

In an optional embodiment, the generator unit is configured to simultaneously or alternatingly generate microwave electromagnetic energy of the first frequency and radiofrequency electromagnetic energy of a third frequency.

The generator unit may include a first generator for generating electromagnetic energy of the first frequency, a second generator for generating electromagnetic energy of the second frequency, and/or a third generator for generating electromagnetic energy of the third frequency. The output of the first generator and the output of the second generator may be combined as described above using a multiplexer.

The output of the third generator may be combined with the output of the multiplexer using a combiner which can include a switch for alternatingly switching between outputting the output of the multiplexer and outputting the output of the third generator. The combiner may include an additional multiplexer for combining the output of the multiplexer and the output of the third generator to simultaneously emit electromagnetic energy of the first frequency, the second frequency, and the third frequency. In this case, microwave sealing and radiofrequency cutting can be simultaneously effected.

If switching between the output of microwave energy and radiofrequency energy is possible, this can be used for sealing the tissue is in microwave energy and then subsequently cutting the tissue using radiofrequency energy. Alternatively, the switch between the output of microwave energy and radiofrequency energy can be executed repeatedly and rapidly providing near simultaneous cutting and sealing.

### BRIEF DESCRIPTION OF THE DRAWINGS

Embodiments of the disclosure are described in detail below with reference to the accompanying drawings, in which:
Fig. 1 shows a schematic view of an embodiment of an electrosurgical apparatus;
Fig. 2 shows a schematic view of a further embodiment of an electrosurgical apparatus;
Fig. 3 shows a perspective view of an embodiment of an electrosurgical instrument (in an open position) of the electrosurgical apparatuses shown in Figs. 1 or 2;
Fig. 4 shows a cross-sectional view of the electrosurgical instrument of Fig. 3 in a closed position (upper image) and a press position (lower image);
Fig. 5 shows a cross-sectional view of a further embodiment of an electrosurgical instrument of the electrosurgical apparatuses shown in Figs. 1 or 2 in a closed position (upper image) and a press position (lower image);
Fig. 6 shows a cross-sectional view of a further embodiment of an electrosurgical instrument of the electrosurgical apparatuses shown in Figs. 1 or 2 in a closed position (upper image) and a press position (lower image);
Fig. 7 shows a perspective view and a cross-sectional perspective view of a first jaw of a further embodiment of an electrosurgical instrument of the electrosurgical apparatuses shown in Figs. 1 or 2;
Fig. 8 shows a cross-sectional perspective view of the first jaw and a first arm of a further embodiment of an electrosurgical instrument of the electrosurgical apparatuses shown in Figs. 1 or 2;
Fig. 9 shows a cross-sectional view of a simulated emission distribution emitted by the first jaw of Fig. 8;
Fig. 10 shows a perspective view of a further embodiment of an electrosurgical instrument (in an open position) of the electrosurgical apparatuses shown in Figs. 1 or 2;
Fig. 11 shows a perspective view of a first jaw of a further embodiment of an electrosurgical instrument of the electrosurgical apparatuses shown in Figs. 1 or 2;
Figs. 12a and 12b show top views of emission distributions emitted by the first jaw of Fig. 11 with Zirconia as a fist isolating portion (Fig. 12a) and with PEEK as the first isolating portion (Fig. 12b);
Fig. 13 shows a cross-sectional perspective view of a further embodiment of an electrosurgical instrument (in a closed position) of the electrosurgical apparatuses shown in Figs. 1 or 2;
Fig. 14 shows a perspective view of the electrosurgical instrument of Fig. 13 in an open position;
Fig. 15 shows a perspective view of a first jaw of a further embodiment of an electrosurgical instrument of the electrosurgical apparatuses shown in Figs. 1 or 2;
Fig. 16 shows a cross-sectional view of a simulated emission distribution emitted by the first jaw of Fig. 15;
Fig. 17 shows a perspective view of a first jaw of a further embodiment of an electrosurgical instrument of the electrosurgical apparatuses shown in Figs. 1 or 2;
Fig. 18 shows a perspective view of a further embodiment of an electrosurgical instrument (in an open position) of the electrosurgical apparatuses shown in Figs. 1 or 2;
Fig. 19 shows a cross-sectional view of a simulated emission distribution emitted by the first jaw of Fig. 18;
Fig. 20 shows a perspective view of a further embodiment of an electrosurgical instrument (in an open position) of the electrosurgical apparatuses shown in Figs. 1 or 2;
Fig. 21 shows a cross-sectional view of the electrosurgical instrument of Fig. 20 in a closed position;
Fig. 22 shows a top view on a first jaw of the electrosurgical instrument of Fig. 20;
Fig. 23 shows a perspective cross-sectional view of a first jaw of a further embodiment of an electrosurgical instrument of the electrosurgical apparatuses shown in Figs. 1 or 2;
Fig. 24 shows a cross-sectional view of a further embodiment of an electrosurgical instrument (in a closed position) of the electrosurgical apparatuses shown in Figs. 1 or 2; and
Fig. 25 shows a cross-sectional view of the embodiment of Fig. 24, wherein the second jaw is further moved towards the first surface relative to the closed position so that the recessed portion is moved closer towards the radiofrequency electrode.

### DETAILED DESCRIPTION; FURTHER OPTIONS AND PREFERENCES

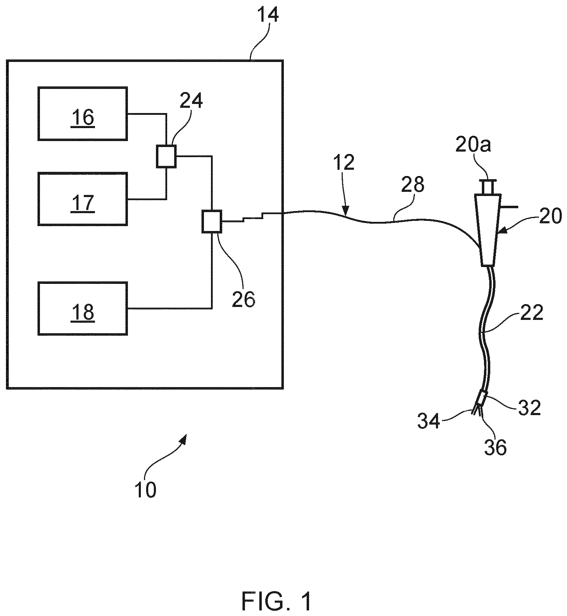

The present disclosure relates to an electrosurgical instrument and apparatus capable of delivering microwave energy to seal tissue (e.g. blood vessels) and of cutting the tissue. The electrosurgical instrument and apparatus may be used in open surgery but may find particular use in procedures where there is restricted access to the treatment site. For example, the electrosurgical instrument of the disclosure may be adapted to fit within the instrument channel of a surgical scoping device i.e. laparoscope, endoscope, or the like. Fig. 1 shows a schematic view of an electrosurgical apparatus 10 in which the electrosurgical instrument of the invention may be used.

Fig. 1 is a schematic diagram of a complete electrosurgical apparatus 10 that is an embodiment of the disclosure. The electrosurgical apparatus 10 is arranged to treat biological tissue using radiofrequency (RF) and/or microwave electromagnetic (EM) energy delivered from an electrosurgical instrument 12. The EM energy emitted by the electrosurgical instrument 12 into a treatment zone can be used to coagulate, cut, and/or ablate tissue in the treatment zone.

The electrosurgical apparatus 10 further comprises a generator unit 14 which can controllably supply radiofrequency and/or microwave electromagnetic energy to the electrosurgical instrument 12. The generator unit 14 may include a first generator 16, a second generator 17, and a third generator 18. Suitable generators for this purpose are described in WO 2012/075844. The generator unit 14 may be arranged to monitor reflected signals received back from the electrosurgical instrument 12 in order to determine an appropriate power level for delivery. For example, the generator unit 14 may be arranged to calculate an impedance seen at the electrosurgical instrument 12 in order to determine an optimal delivery power level.

The electrosurgical apparatus 10 further comprises a surgical scoping device 20, such as a bronchoscope, endoscope, gastroscope, laparoscope or the like. The scoping device 20 may include a handpiece 20a and a flexible shaft 22. The handpiece 20a may include means for guiding the flexible shaft 22 through a cavity of a body. For example, the handpiece 20a can include means for moving a distal end of the flexible shaft 22 to change direction of the distal end of the flexible shaft 22. This helps manoeuvring the flexible shaft 22 through the cavity of the body. The flexible shaft 22 may include a working channel through which elongated structures can be moved and, thus, positioned at the treatment zone within the cavity of the body.

The first generator 16, the second generator 17, and the third generator 18 are each configured to generate electromagnetic energy of a fixed frequency. However, the generator unit 14 is not limited thereto; the first generator 16, the second generator 17, and/or the third generator 18 can be configured to generate AC electromagnetic energy in a continuous range between a minimum frequency and a maximum frequency. The frequency of the electromagnetic energy to be generated by the first generator 16, the second generator 17, and/or the third generator 18 may be selected using an interface (not shown in the figures).

The generator unit 14 can include a multiplexer 24 which is electrically connected or coupled to the first generator 16 and to the second generator 17. The multiplexer 24 combines the outputs of the first generator 16 and the second generator 17 and outputs the combined outputs to a combiner 26. The multiplexer 24 may include a diplexer.

The combiner 26 is configured to temporally switch between outputting the output of the multiplexer 24 or the output of the third generator 18. The combiner 26 may also be configured to combine the outputs of the multiplexer 24 and of the third generator 18. In this case, the combiner 26 acts as a diplexer.

The generator unit 14 is thus capable of generating and controlling power to be delivered to the electrosurgical instrument 12, e.g. via a transmission line 28, which extends from the generator unit 14 through the surgical scoping device 20 and instrument channel to the distal tip of the instrument channel. The generator unit 14 may have a user interface for selecting and/or controlling the power delivered to the electrosurgical instrument 12, e.g. controlling the first to third generators 16, 17, 18 and/or the combiner 26. The generator unit 14 may have a display for showing the selected energy delivery mode. In some examples, the generator unit 14 may allow for an energy delivery mode to be selected based on the size of the vessel to be sealed.

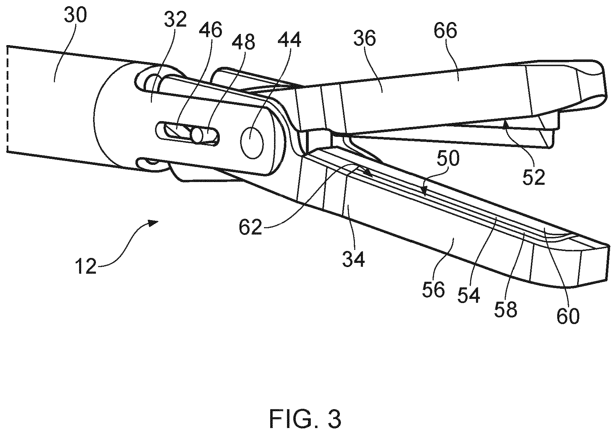

The electrosurgical instrument 12 can include the transmission line 28, an instrument shaft 30, a joint 32, a first jaw 34, and/or a second jaw 36 which will be discussed in further detail in connection with Fig. 3. The transmission line 28 may include a single coaxial cable that connects the generator unit 14 to the first jaw 34 and/or second jaw 36 for conveying the radiofrequency and/or microwave energy.

### first electrode

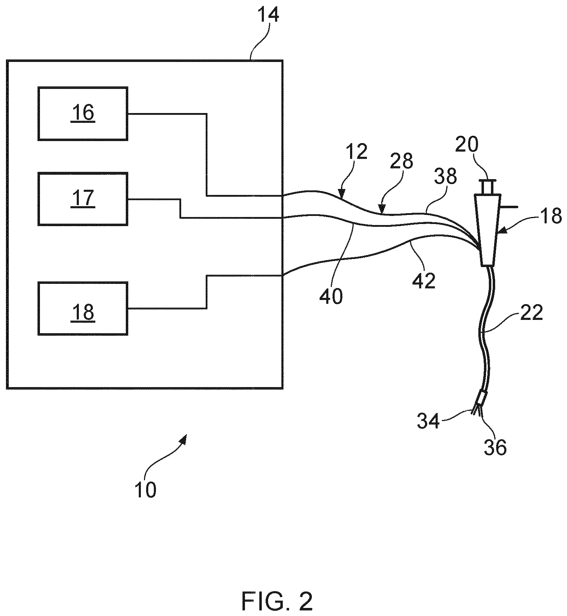

The electrosurgical apparatus 10 of Fig. 2 includes the same features and characteristics as the electrosurgical apparatus 10 of Fig. 1 except for the following differences.

The generator unit 14 does not include the multiplexer 24 and the combiner 26. Further, the transmission line 28 includes a first coaxial cable 38 that is directly connected to the first generator 16, a second coaxial cable 40 that is directly connected to the second generator 17, and a third cable 42 that is directly connected to the third generator 18. Thus, the first coaxial cable 38 and the second coaxial cable 40 are configured to convey microwave energy while the third cable 42 is configured to convey radiofrequency energy. The third cable 42 can include one or more wires. Thus, in this embodiment, the application of microwave energy and radiofrequency energy can be controlled by turning on and off the first to third generators 16, 17, and 18.

Fig. 3 shows a schematic perspective view of a distal end of an embodiment of the electrosurgical instrument 12. The first jaw 34 and the second jaw 36 are rotatably or pivotally connected or coupled to the instrument shaft 30 which is dimensioned to fit within the instrument channel of the surgical scoping device 20. The instrument shaft 30 comprises a tubular sheath that covers the transmission line 28 for carrying microwave and/or radiofrequency energy to the jaws 34, 36 together with various control wires or (actuation) rods that are arranged to control and/or physically manipulate the first and second jaws 34, 36, as discussed below.

The first jaw 34 and the second jaw 36 are operably coupled to the joint 32 that is mounted on a distal end of the instrument shaft 30. Both the first and second jaws 34, 36 may be arranged to pivot relative to the joint 32. The joint 32 may be arranged to ensure that the jaws remain laterally aligned as they are moved together.

In an alternative embodiment, the pair of jaws 34, 36 comprises a static jaw that is fixed relative to the instrument shaft 30 or the joint 32. The other jaw is pivotable or rotatable.

In the embodiment shown, the joint 32 includes a pivot axle 44 which defines a pivot axis. The first jaw 34 and the second jaw 36 can pivot around the pivot axis or pivot axle 44. For example, the pivot axle 44 is fixed to the joint 32 and the first jaw 34 and the second jaw 36 can rotate around the pivot axle 44.

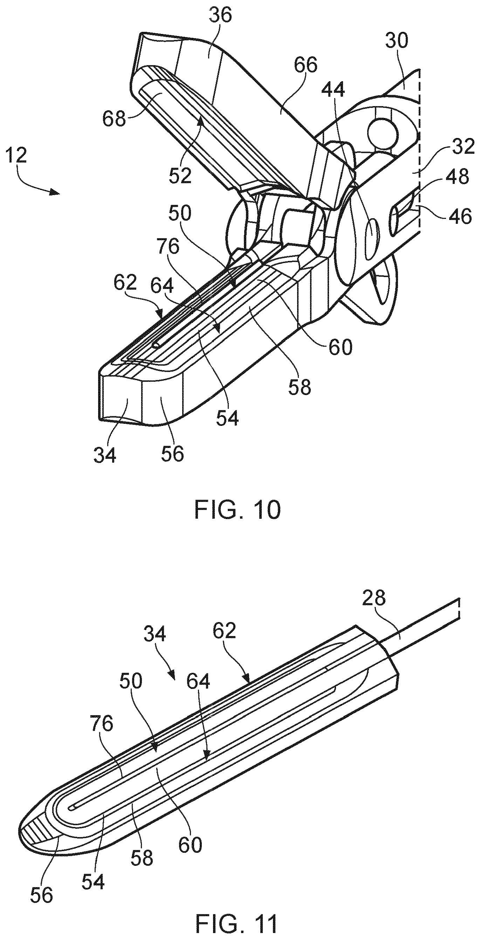

The joint 32 may have a clevis structure and slots 46 on the arms of the clevis structure. The slot 46 may have an elongate shape and can be a through-hole or a recess in an inner surface of the clevis structure. The first jaw 34 and/or the second jaw 36 also include elongated slots (see Fig. 10). A control wire or actuation rod includes a cam 48 which is inserted in the slots 46 of the clevis structure and in the slots of the first jaw 34 and the second jaws 36. The engagement of the cam 48 with the slots provides an actuation mechanism which translates a back-and-forth movement of the control wire (and thus of the cam 48) into a rotational movement of the first jaw 34 and the second jaw 36 around the pivot axle 44.

In use, the first jaw 34 and the second jaw 36 are intended to grip biological tissue (in particular a blood vessel) therebetween. The first jaw 34 and the second jaw 36 are arranged to apply pressure to the biological tissue between the opposed surfaces of the jaws 34, 36 and deliver energy (preferably microwave and/or radiofrequency electromagnetic energy) into the tissue from the transmission line 28.

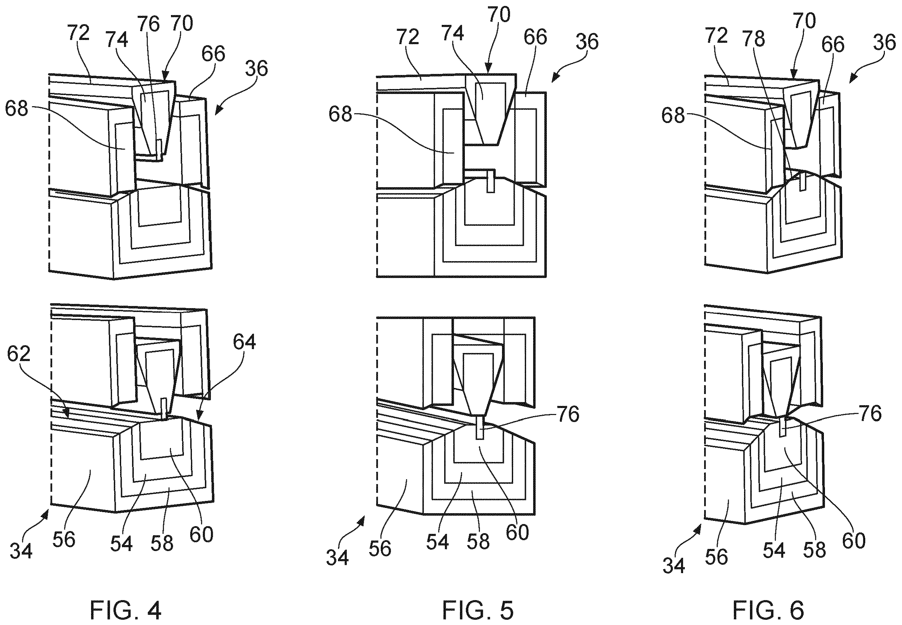

The first jaw 34 includes a first surface 50 which opposes a second surface 52 of the second jaw 36. The first surface 50 and/or the second surface 52 may form an outer surface of the first jaw 34 and the second jaw 36 respectively, which can be brought into contact with each other when the jaws 34, 36 are in the closed position. For example, the first surface 50 and the second surface 52 may be considered pressure pads or pressure areas with which pressure can be applied to the tissue grasped between the first jaw 34 and the second jaw 36 (see for example Figs. 4 to 6 which show the closed position of the first jaw 34 and the second jaw 36).

In the embodiment of Figs. 3 and 4, the first jaw 34 includes a first electrode 54, a second electrode 56, a first isolating portion 58, and/or a second isolating portion 60. The first electrode 54 and the second electrode 56 are made from an electrically conductive material, such as metal or a metal alloy. The second electrode 56 may form the outer surface of the first jaw 34 and/or may provide the connection to the pivot axle 44. Further, the slot of the first jaw 34 may be provided in the second electrode 56. Thus, the second electrode 56 may have a function of providing the stability of the first jaw 34.

The second electrode 56 may have a form of a half-shell in a portion along the first surface 50. The second electrode 56 may surround the first electrode 54, the first isolating portion 58 and/or the second isolating portion 60. This means that the first electrode 54, the first isolating portion 58, and/or the second isolating portion 60 may be embedded in the half-shell of the second electrode 56. The shape of the second electrode 56 may also be considered as providing a recess or channel in which the first electrode 54, the first isolating portion 58 and/or the second isolating portion 60 are arranged.

The first isolating portion 58 electrically isolates the first electrode 54 from the second electrode 56. The first isolating portion 58 may be made from an electrically non-conductive material such as a ceramic material (e.g. including Zirconia), PEEK, silicon, and/or other plastic materials. The first isolating portion 58 may be sandwiched between the first electrode 54 and the second electrode 56.

The first electrode 54 and the second electrode 56 may have a U-shape or V-shape (see Fig. 4) in a cross-sectional view along a section of the first jaw 34. Further, the first electrode 54 and the second electrode 56 may have a plate-shape (see Fig. 4) along this section of the first jaw 34. At a distal end of the first jaw 34, the first electrode 54 and/or the second electrode 56 may have a different configuration so that the first electrode 54 and/or the second electrode 56 do not have an open end but a closed end. This means that the second electrode 56 can shield the first electrode 54 in all directions away from the first surface 50.

The first electrode 54 and/or the second electrode 56 are exposed at the first surface 50 to allow for contact with the tissue clamped between the first jaw 34 and the second jaw 36. In particular, two sections of the first electrode 54 and the second electrode 56 are exposed on the first surface 50 which are spaced from each other by a gap. In the embodiment shown, the two exposed sections are straight and extend in a direction of extension of the first jaw 34. Each exposed section includes an exposed section of the first electrode 54, the second electrode 56, and the first isolating portion 58. The two exposed sections provide a first sealing area 62 and the second sealing area 64 which are separated from each other by the gap in a direction perpendicular to the extension of the first jaw 34.

The first sealing area 62 and the second sealing area 64 are each provided for the emission of microwave energy so that tissue is sealed at the first sealing area 62 and the second sealing area 64. In particular, the intensity of the microwave energy that is emitted from the first electrode 54 and the second electrode 56 is highest at the sealing areas 62 and 64. The intensity of the emitted microwave energy is considerably lower in the area of the gap (i.e. between the first sealing area 62 and the second sealing area 64) compared to the first sealing area 62 and the second sealing area 64, for example 10% to 80% less than over the first sealing area 62 and the second sealing area 64. This results in that tissue is effectively sealed at the first sealing area 62 and at the second sealing area 64 but not in the gap therebetween.

The first sealing area 62 and the second sealing area 64 may be provided by the end faces of the plate-shaped first electrode 54, the second electrode 56, and the first isolating portion 58. The exposed sections of the first electrode 54, the second electrode 56, and the first isolating portion 58 outside the first and second sealing areas 62 and 64 may form a loop at the first surface 50 which connects the first sealing area 62 with the second sealing area 64 on the first surface 50. This loop may not be considered belonging to the first sealing area 62 or the second sealing area 64 since there is no separation by a gap.

The exposed sections of the first electrode 54 and second electrode 56 over the first sealing area 62 and the second sealing area 64 may form two parallel straight lines which are respectively separated by the gap.

In the embodiment of Figs. 3 and 4, the separation of the first sealing area 62 and the second sealing area 64 by the gap is provided by the second isolating portion 60. The second isolating portion 60 separates the exposed sections of the first electrode 54 on the first surface 50. In other words, the second isolating portion 60 separates the first sealing area 62 from the second sealing area 64. The second isolating portion 60 may be received in a cavity formed by the U-shaped first electrode 54. In other words, the first electrode 54 forms a channel similar to the second electrode 56. The second isolating portion 60 may be embedded or arranged in this channel.

The second isolating portion 60 may have a flat or straight outer surface at the first surface 50. For example, the exposed section of the second isolating portion 60 on the first surface 50 may be parallel to a lower surface of the second electrode 56 defining a lower surface of the first jaw 34 (see Fig. 4). The first sealing area 62 and the second sealing area 64 may be inclined relative to each other and relative to the exposed section of the second isolating portion 60. The angle between a plane defined by the exposed section of the second isolating portion 60 and a plane defined by the first and/or second sealing surface 62, 64 may be between 5° to 45°. Optionally, the first sealing area 62 and the second sealing area 64 define the same angle with the exposed section of the second isolating portion 60 at the first surface 50.

The inclined arrangement of the first sealing area 62 and the second sealing area 64 increases the respective surface areas without widening the first jaw 34. Further, after cutting the tissue clamped between the first jaw 34 and the second jaw 36, the cut tissue can be more easily released from the first jaw 34 compared to a flat (i.e. non-inclined) first surface 50.

The second isolating portion 60 may comprise a silicone-based material or silicon. Other examples include polyimide, PTFE or FEP type materials. These types of materials are more flexible or softer compared to a ceramic material which helps to reduce the pressure on the tissue over the area of the gap (i.e. between the first sealing area 62 and the second sealing area 64). So the pressure on the tissue can be reduced in an area where the tissue is not sealed. In other words, the first jaw 34 and the second jaw 36 press and seal the tissue at the first sealing area 62 and the second sealing area 64. In an embodiment, the first jaw 34 and the second jaw 36 press and seal the tissue only at the first sealing area 62 and the second sealing area 64.

The second jaw 36 includes a fourth electrode 66, a fourth isolating portion 68, and/or a first arm 70.

The fourth electrode 66 is made from an electrically conductive material, such as metal. The fourth electrode 66 may form the outer surface of the second jaw 36 and/or may provide the connection to the pivot axle 44. Further, the slot of the second jaw 36 may be arranged on the fourth electrode 66. Thus, the fourth electrode 66 may have a function of providing the stability of the second jaw 36. The fourth electrode 66 may have a form of a half-shell in a portion of the second surface 52. The fourth electrode 66 may surround the fourth isolating portion 68. This means that the fourth isolating portion 68 may be embedded in the half-shell of the fourth electrode 66. The shape of the fourth electrode 66 may also be considered as providing a recess or channel in which the fourth isolating portion 68 is arranged.

The fourth electrode 66 may be mirror-symmetric to the second electrode 56. The fourth electrode 66 may be a U-shape (see Fig. 4) in a cross-sectional view along a section of the second jaw 36. Further, the fourth electrode 66 may have a plate-shape (see Fig. 4) along this section of the second jaw 36. At a distal end of the second jaw 36, the fourth electrode 66 may have a different configuration so that fourth electrode 66 does not have an open end but a closed end. This means that fourth electrode 66 can shield the first electrode 54 in all directions away from the second surface 52. The second electrode 56 and the fourth electrode 66 may be electrically connected to each other, either directly or by being connected to the same conductor of the transmission line 28.

The fourth isolating portion 68 is made from an electrically non-conductive material, optionally a silicone-based material or silicon. Other examples include polyimide, PTFE or FEP type materials. The sections of the fourth electrode 66 and of the fourth isolating portion 68 that are exposed to contact tissue in the closed position may define or form the second surface 52. Thus, tissue can be grasped between the first electrode 54 and the second electrode 56 on the first jaw 34 and the fourth electrode 66 on the second jaw 36.

The first arm 70 may include an arm support 72, a third isolating portion 74, and/or a third electrode 76. The arm support 72 may provide the stability of the first arm 70, can be made from an electrically conductive material, such as metal, and/or may be connected to the pivot axle 44. Further, a control wire may be coupled to the first arm 70, optionally the arm support 72, for rotating the first arm 70 relative to the second jaw 36 and/or around the pivot axle 44.

The fourth electrode 66 and the fourth isolating portion 68 may each include an opening or through-hole through which the first arm 70 can be moved. The dimensions and/or the shape of the opening may match the ones of the first arm 70 so that, when the second jaw 36 and the first arm 70 are arranged at the same angular position (i.e. the first arm 70 is in line with the second jaw 36), there is a small gap or a sliding contact between the outer surface of the first arm 70 and the inner surface of the opening. In particular, the arm support 72 and the fourth electrode 66 may form a continuous half-shell in this position so that the arm support 72 forms a part of the shielding function of the second electrode 56. The arm support 72 and the fourth electrode 66 may be electrically connected to each other, either directly or by being connected to the same conductor of the transmission line 28.

The third electrode 76 is configured to emit radiofrequency energy for tissue cutting and is electrically isolated from the fourth electrode 66 and/or the first electrode 54. The third electrode 76 may be an active electrode for delivering radiofrequency electromagnetic energy while the first electrode 54, the second electrode 56, and/or the third electrode 76 are return electrodes. The third electrode 76 is connected to the transmission line 28, and optionally to the cable 42. The third electrode 76 may be an example of a radiofrequency electrode.

The third electrode 76 is exposed on the third isolating material 74 which in turn is attached to the arm support 72. The third electrode 76 is arranged to first contact tissue that is clamped between the first jaw 34 and the second jaw 36 if the first arm 70 is brought into contact with the tissue. In other words, the third electrode 76 protrudes from the third isolating portion 74 in a direction towards the first jaw 34. The third electrode 76 may be a ridge or bar made from an electrically conductive material, such as a metal or a metal alloy. The third isolating portion 74 is made from an electrically non-conductive material, such as a ceramic or plastic material.

Due to the attachment of the first arm 70 to the pivot axle 44, the first arm 70 may be freely moved relative to the second jaw 36. This can be used to reduce pressure between the third electrode 76 and the second isolating portion 60 in the closed position. As shown in Fig. 4 (upper image), the first jaw 34 and the second jaw 36 are moved towards each other to clamp tissue therebetween (not shown in Fig. 4), i.e. Fig. 4 (upper image) shows the closed position of the first jaw 34 and the second jaw 36. In this closed position, the first arm 70 may not be actuated or can move freely so that the first arm 70 does not apply pressure to the tissue and is slightly offset to the second jaw 36 (see Fig. 4). In this configuration, the tissue may be sealed using microwave energy.

Subsequently, the first arm 70 is actuated to be pressed against the second isolating portion 60 forming a press position (lower image of Fig. 4). In the press position, the third electrode 76 emits radiofrequency energy to cut the tissue. This means the pressure on the tissue between the third electrode 76 and the second isolating portion 60 can be selectively increased during radiofrequency cutting compared to the pressure applied during microwave sealing. At the same time of radiofrequency cutting or after completion of the radiofrequency cutting, the first jaw 34 and the second jaw 36 may be moved away from the closed position (lower image of Fig. 4) to un-clamp the tissue allowing the cut tissue to be released. The inclination of the first sealing area 62 and the second sealing area 64 improve the release of the cut tissue.

The embodiment of the electrosurgical instrument 12 shown in Fig. 5 includes the same features, characteristics, and/or optional embodiment as the embodiment of the electrosurgical instrument 12 shown in Figs. 3 and 4 except for the following differences.

The third electrode 76 is not arranged on the first arm 70 but on the second isolating portion 60. The third electrode 76 protrudes from the second isolating portion 60 towards the second jaw 36. A contact surface of the first arm 70 (i.e. the surface of the first arm 70 that is in contact with the tissue clamped between the first jaw 34 and the second jaw 36) is provided by the third isolating portion 74.

The functionality of the electrosurgical instrument 12 of the embodiment of Fig. 5 is similar to the functionality of embodiment of the electrosurgical instrument 12 shown in Figs. 3 and 4. The first arm 70 is used to control the pressure applied to tissue between the third electrode 76 and the first arm 70, especially the third isolating portion 74. As described above, pressure on the tissue between the third electrode 76 and the first arm 70 can be varied by moving the first arm 70 towards and away from the first jaw 34 as depicted in Fig. 5.

The embodiment of the electrosurgical instrument 12 shown in Fig. 6 includes the same features, characteristics, and/or optional embodiment as the embodiment of the electrosurgical instrument 12 shown in Fig. 5 except for the following differences.

The third electrode 76 is again not arranged on the first arm 70 but on the second isolating portion 60. The third electrode 76 again protrudes from the second isolating portion 60 towards the second jaw 36. A contact surface of the first arm 70 (i.e. the surface of the first arm 70 that is in contact with the tissue clamped between the first jaw 34 and the second jaw 36) is however not provided by the third isolating portion 74. The third isolating portion 74 is replaced by a component having the same shape and dimensions as the third isolating portion 74 but is made from an electrically conductive material, such as metal. In this embodiment, the arm support 72 and the portion corresponding to the third isolating portion 74 can form a unitary or single component made from an electrically conductive material.

In the press position of the first arm 70, there would be an electrical connection between the third electrode 76 and the portion corresponding to the third isolating portion 74. To prevent this, the fourth isolating portion 68 includes a membrane section 78 which covers the opening through which the first arm 70 is movable. This membrane section 78 is deformable and electrically isolates the third electrode 76 from the first arm 70, in particular the portion corresponding to the third isolating portion 74.

The membrane section 78 is deformed in the closed position as the third electrode 76 pushes the membrane section 78 into the opening. Similarly, the membrane section 78 can be deformed by pushing the first arm 70 through the opening towards the first jaw 34. The flexibility or softness of the membrane section 78 can be achieved by making the fourth isolating portion 68 from a silicon-based material or silicon and/or by making the membrane section 78 thin. The membrane section 78 and the rest of the fourth isolating portion 68 can form a unitary or single component.

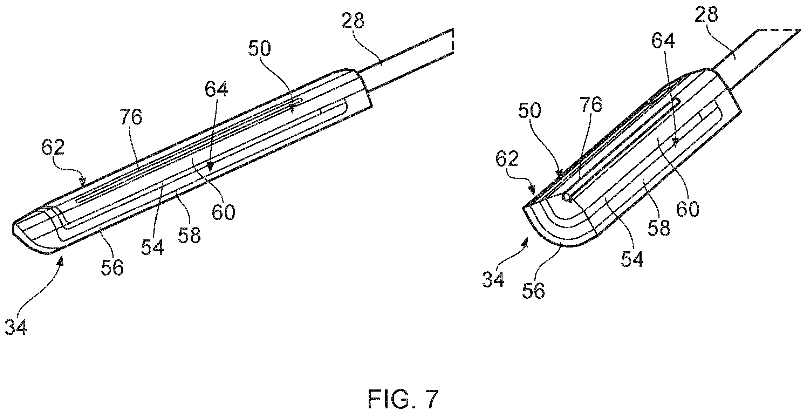

The embodiment of the electrosurgical instrument 12 shown in Fig. 7 includes the same features, characteristics, and/or optional embodiment as the embodiments of the electrosurgical instrument 12 shown in Figs. 5 or 6 except for the following differences.

The section of the second isolating portion 60 that is exposed at the first surface 50 is not flat as with the embodiments shown in Figs. 5 or 6 but is also inclined. In the embodiment of Fig. 7, one half of the section of the second isolating portion 60 that is exposed at the first surface 50 is flush with the first sealing area 62 while another half of the section of the second isolating portion 60 that is exposed at the first surface 50 is flush with the second sealing 64. The respective sections of the second isolating portion 60 that are exposed at the first surface 50 provide a flat area with the first sealing area 62 and the second sealing area 64. In other words, the first surface 50 is divided by the third electrode 76 into two flat areas which are angled to each other. For example, these two areas are mirror-symmetric to the third electrode 76.

The plate-shaped portions of the first electrode 54 and the second electrode 56 have a shape of a circle segment in a cross-sectional view of the first jaw. Further, Fig. 7 shows where the transmission line 28, e.g. the first coaxial cable 38 is connected to the first jaw 34. This arrangement may also be used with the embodiments of Figs. 3 to 6.

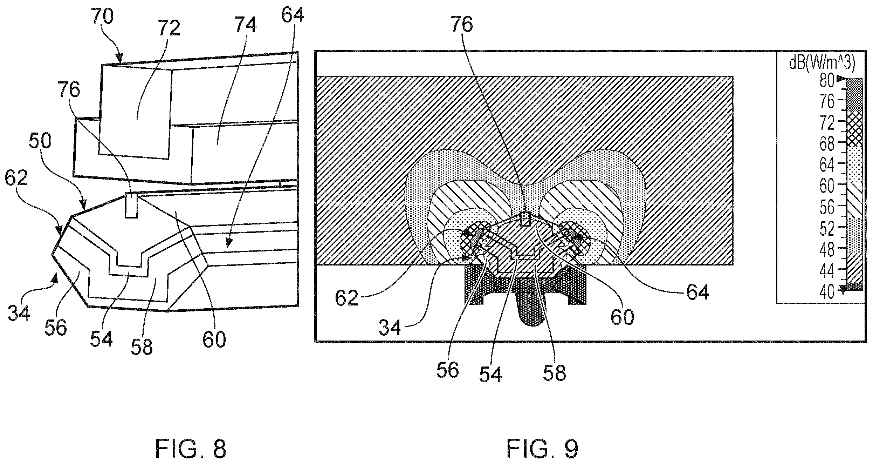

The embodiment of the electrosurgical instrument 12 shown in Fig. 8 includes the same features, characteristics, and/or optional embodiment as the embodiments of the electrosurgical instrument 12 shown in Figs. 5 to 7 except for the following differences.

The section of the second isolating portion 60 that is exposed at the first surface 50 is not flat as with the embodiments shown in Figs. 5 or 6 but is also inclined as with the embodiment of Fig. 7. In the embodiment of Fig. 8, one half of the section of the second isolating portion 60 that is exposed at the first surface 50 is arranged at a different angle with respect to the first sealing area 62 while another half of the section of the second isolating portion 60 that is exposed at the first surface 50 is arranged at a different angle with respect to the second sealing area 64. The respective sections of the second isolating portion 60 that are exposed at the first surface 50 provide a flat area that are angled with respect to the first sealing area 62 and the second sealing area 64, respectively. In other words, the first surface 50 is divided into four flat areas which are angled to each other. For example, these four areas are mirror-symmetric to the third electrode 76.

It is possible that any one of the above describes the surface areas of the first surface 50 (e.g. the first sealing area 62, the second sealing area 64, and/or the one or more sections of the second isolating portion 60 that is exposed at the first surface 50) are not flat but curved or arc-shaped. In this case, the degree of curvature may differ amongst these sections.

The plate-shaped portion of the first electrode 54 includes, in a cross-sectional view of the first jaw 34, a V-shape with an additional U-shape in the middle of the V-shape. The second electrode 56 is not entirely plate-shaped but includes a varying thickness in a cross-sectional view of the first jaw 34. This may be done for providing a constant distance between the first electrode 54 and the second electrode 56 while increasing strength of the second electrode 56 by locally increasing its thickness. However, other means are possible for providing a constant distance between the first electrode 54 and the second electrode 56.

The third isolating portion 74 surrounds the arm support 72 in the embodiment of Fig. 8. Thus, a width of the third isolating portion 74 is greater than a width of the arm support 72, which is the opposite case to the embodiments of Figs. 3 to 4. This may be used for electrically isolating the arm support 72 from the fourth electrode 66.

Fig. 9 shows the distribution of microwave energy emitted by the first electrode 54 and the second electrode 56 of the embodiment of Fig. 8. It is readily apparent that the intensity of the emitted microwave energy is highest above the first sealing area 62 and the second sealing area 64 whereas little or no microwave intensity is present at the third electrode 76. So, tissue in contact with the third electrode 76 is not sealed during the emission of microwave energy. Non-sealed tissue can be more effectively cut using radiofrequency energy. So, the embodiments of the electrosurgical instrument 12 discussed so far provide a spatial separation of the microwave sealing and the radiofrequency cutting by physically separating the first sealing area 62 and the second sealing area 64 (formed from the first electrode 54 and the second electrode 56) from the third electrode 76.

The embodiment of the electrosurgical instrument 12 shown in Fig. 10 includes the same features, characteristics, and/or optional embodiment as the embodiments of the electrosurgical instrument 12 shown in Figs. 5 to 8 except for the following differences.

The second jaw 36 does not include the first arm 70. Instead, the fourth electrode 66 provides a complete half-shell which is completely filled with the fourth isolating portion 68, i.e. no third isolating portion 74 is present. The fourth isolating portion 68 and the fourth electrode 66 define the second surface 52. In the closed position, the fourth isolating portion 68 is in contact with the third electrode 76. The fourth isolating portion 68 is, as explained above, soft so that, in the closed position, pressure on the tissue between the fourth isolating portion 68 and the third electrode 76 can be reduced, i.e. is smaller compared to the pressure that is applied between the fourth electrode 66 and the first sealing area 62 as well as the second sealing area 64. The section of the fourth isolating portion 68 that is exposed on the second surface 52 is complementary to the shape of the first surface 50. In general, the shape of the second surface 52 is complementary (i.e. matches) the shape of the first surface 50. This configuration of the shape of the second surface 52 may apply to all embodiments discussed herein.

The embodiment of the electrosurgical instrument 12 shown in Fig. 11 includes the same features, characteristics, and/or optional embodiment as the embodiments of the electrosurgical instrument 12 shown in Figs. 5 to 9 except for the following differences.

The first sealing area 62 and the second sealing area 64 are flat and are not inclined to each other. For example, the first sealing area 62 and the second sealing area 64 are parallel to a bottom surface of the first jaw 34. The second isolating portion 60 may protrude from the first sealing area 62 and the second sealing area 64. The third electrode 76 may protrude from the second isolating portion 60.

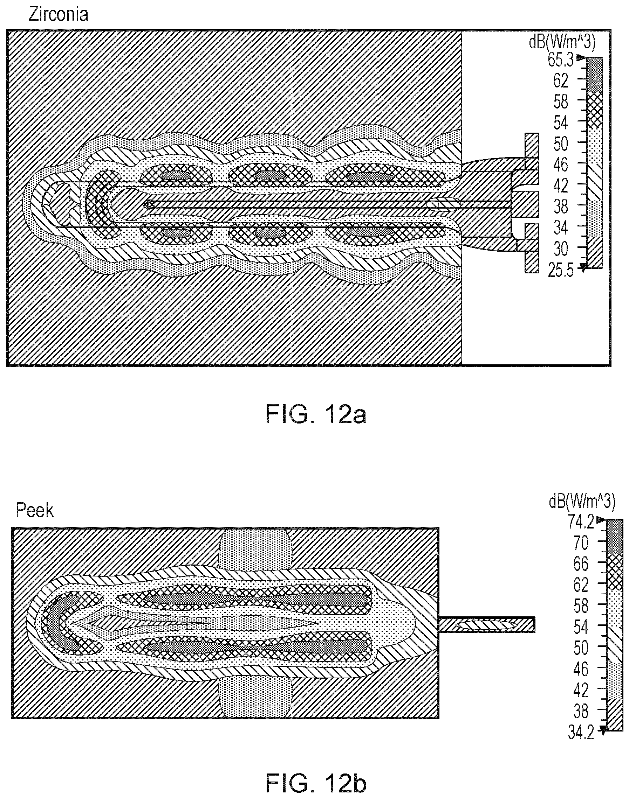

Figs. 12a and 12b show emission distributions of the microwave energy if the first isolating portion 58 is made from Zirconia (Fig. 12a) and PEEK (Fig. 12b). The dielectric properties of the respective materials affect the microwave emission characteristics. It is apparent that the emission distributions include minima and maxima of intensity along the extension of the exposed sections of the first electrode 54 and the second electrode 56. This effect is more pronounced with Zirconia compared to Peek which shows a more even emission distribution.

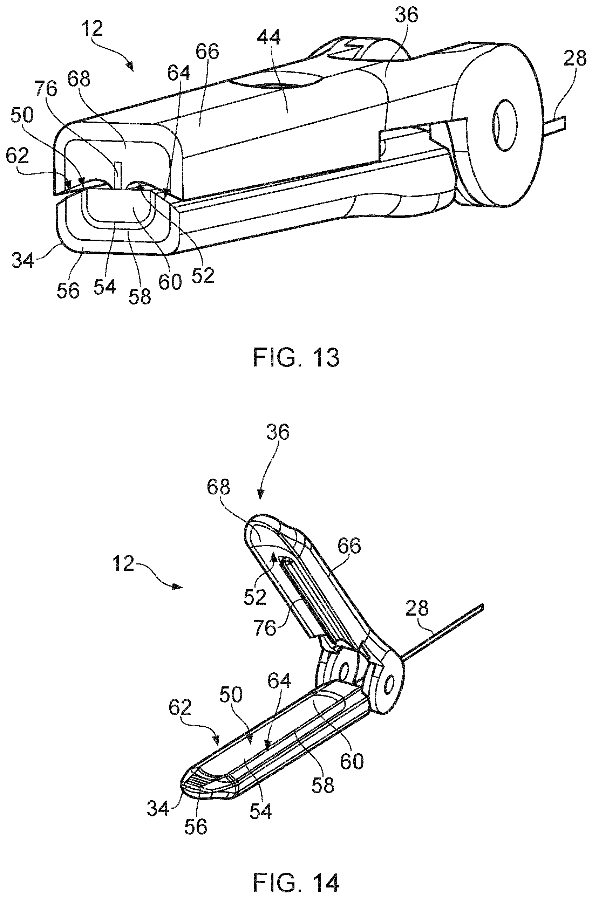

The embodiment of the electrosurgical instrument 12 shown in Figs. 13 and 14 includes the same features, characteristics, and/or optional embodiment as the embodiments of the electrosurgical instrument 12 shown in Fig. 10 except for the following differences.

The third electrode 76 is arranged on the second jaw 36 in contrast to the embodiment shown in Fig. 10 where the third electrode 76 is arranged on the first jaw 34. Seen differently, the embodiment of Fig. 13 corresponds to the embodiment of Figs. 3 and 4 but without the first arm 70. The third electrode 76 protrudes from the fourth isolating portion 68 towards the first jaw 34. The fourth isolating portion 68 has, in a cross-sectional view, no flat surface but an arc shape that spans from the third electrode 76 to the exposed section of the fourth electrode 66.

Further, the second isolating portion 60 is made from a silicon-based material or silicon. The second isolating portion 60 is soft or flexible due to chosen material so that, in the closed position, pressure on the tissue between the second isolating portion 60 and the third electrode 76 can be reduced, i.e. is smaller compared the pressure that is applied between the fourth electrode 66 and the first sealing area 62 as well as the second sealing area 64.

The first sealing area 62 and the second sealing area 64 are inclined while the exposed section of the second isolating portion 60 is flat as described in connection with the embodiments of Figs. 3 to 6.

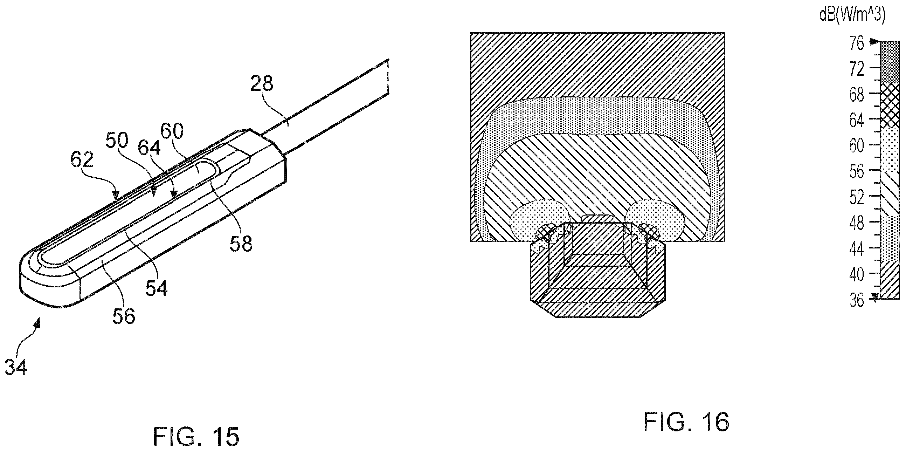

The embodiment of the electrosurgical instrument 12 shown in Figs. 15 and 16 includes the same features, characteristics, and/or optional embodiment as the embodiments of the electrosurgical instrument 12 shown in Figs. 13 and 14 to except for the following differences.

The corners of the plate-shaped portions of the first electrode 54, the second electrode 56, the first isolating portion 58, and/or the second isolating portion 60 form a rectangle in a cross-sectional view in contrast to the embodiment of Figs. 13 and 14 where the corners are rounded in a cross-sectional view. The second isolating portion 60 has a rectangular shape in a cross-sectional view.

Fig. 16 shows the distribution of microwave energy emitted by the first electrode 54 and the second electrode 56 of the embodiment of Fig. 15. It is readily apparent that the intensity of the emitted microwave energy is highest above the first sealing area 62 and the second sealing area 64 whereas little or no microwave intensity is present above the second isolation portion 60, i.e. in the area of the gap. So, tissue positioned between the third electrode 76 and the second isolation portion 60 is not sealed during the emission of microwave energy. Non-sealed tissue can be more effectively cut using radiofrequency energy. So, the embodiment of the electrosurgical instrument 12 of Figs. 13 to 15 provide a spatial separation of the microwave sealing and the radiofrequency cutting by physically separating the first sealing area 62 and the second sealing area 64 (defined by the respective exposed sections of the first electrode 54 and the second electrode 56) from the third electrode 76 in the closed position.

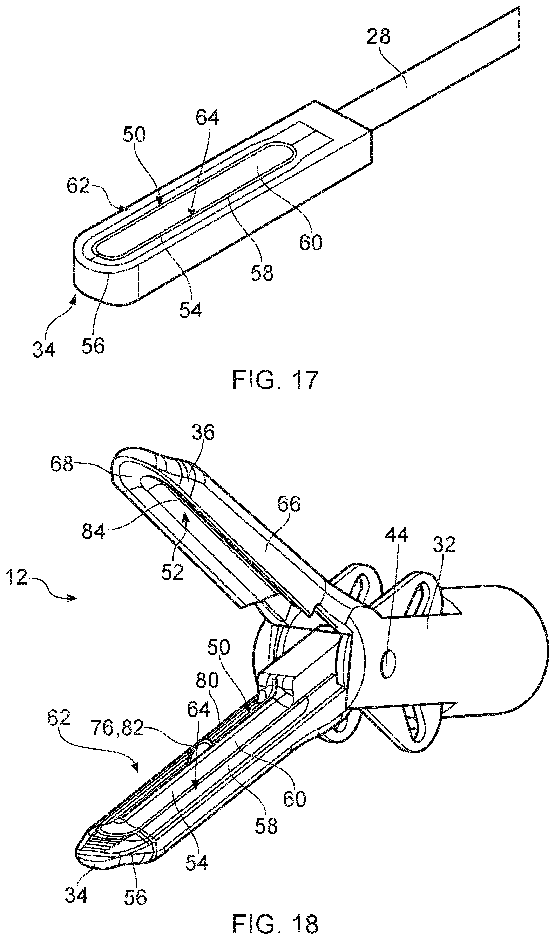

The embodiment of the electrosurgical instrument 12 shown in Fig. 17 includes the same features, characteristics, and/or optional embodiment as the embodiments of the electrosurgical instrument 12 shown in Figs. 15 and 16 except for the following differences.

The first sealing area 62, the second sealing area 64, and the exposed section of the second isolating portion 60 are flat, i.e. the first sealing area 62, the second sealing area 64, and the exposed section of the second isolating portion 60 define an angle of 180° with each other and/or are parallel to each other. In other words, the first surface 50 is flat.

The embodiment of the electrosurgical instrument 12 shown in Fig. 18 includes the same features, characteristics, and/or optional embodiment as the embodiments of the electrosurgical instrument 12 shown in Fig. 10 except for the following differences.

The second isolating portion 60 includes a first groove 80 in which a blade 82 is movably arranged. The blade 82 can be moved within the first groove 80 by a control wire or actuation rod not shown in Fig. 18. The blade 82 also serves as the third electrode 76, i.e. is configured to emit radiofrequency energy. The blade 82 may have a cutting edge so that the blade 82 may mechanically cut tissue as well as by emitting radiofrequency energy. The extension of the first groove 80 defines the cutting line. Here again, the cutting line or the first groove 80 is spatially separated from the first sealing area 62 and the second sealing area 64. Thus, the electrosurgical instrument 12 shown in Fig. 18 also provides a spatial separation of (radiofrequency) cutting and microwave sealing.

The fourth isolating portion 68 is made from a rigid and/or electrically non-conductive material, such as ceramic and/or a plastic material. The fourth isolating portion 68 includes a second groove 84 which is aligned with the first groove 80 so that the blade 82 can move within the second groove 84 in the closed position. This means the blade 82 is positioned in the first groove 80 and in the second groove 84 in the closed position. In the open position, the blade 82 is only arranged in the first groove 80. The first groove 80 may act as a guide rail for the blade 82.

The actuation mechanism for opening and closing the first jaw 34 and the second jaw 36 differs from the actuation mechanism described above. The slot 46 is not open as with the embodiment shown in Fig. 10 but closed. So, the slot 46 is a recess or slit in an inner surface of the arms of the clevis structure of the joint 32.

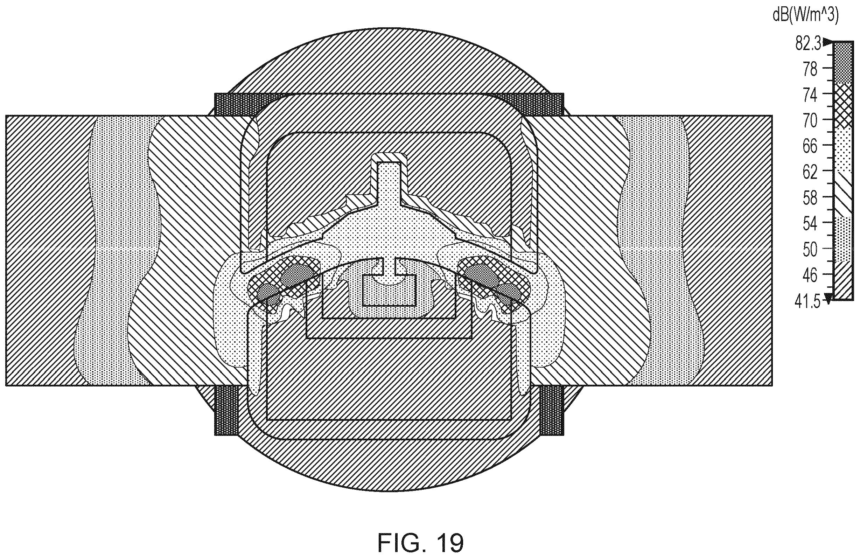

Fig. 19 shows the distribution of microwave energy emitted by the first electrode 54 and the second electrode 56 of the embodiment of Fig. 18. It is readily apparent that the intensity of the emitted microwave energy is highest above the first sealing area 62 and the second sealing area 64 whereas little microwave intensity is present above the second isolation portion 60, especially over the first groove 80 in which the blade 82 is movable (not shown in Fig. 19). So, tissue positioned between the second isolation portion 60, especially the first groove 80, and the fourth isolating portion 68, especially the second groove 84, is not sealed during the emission of microwave energy. Non-sealed tissue can be more effectively cut using radiofrequency energy. So, the embodiment of the electrosurgical instrument 12 of Fig. 18 provides a spatial separation of the microwave sealing and the radiofrequency cutting by physically separating the first sealing area 62 and the second sealing area 64 (defined by the respective exposed sections of the first electrode 54 and the second electrode 56) from the third electrode 76 or blade 82 in the closed position.

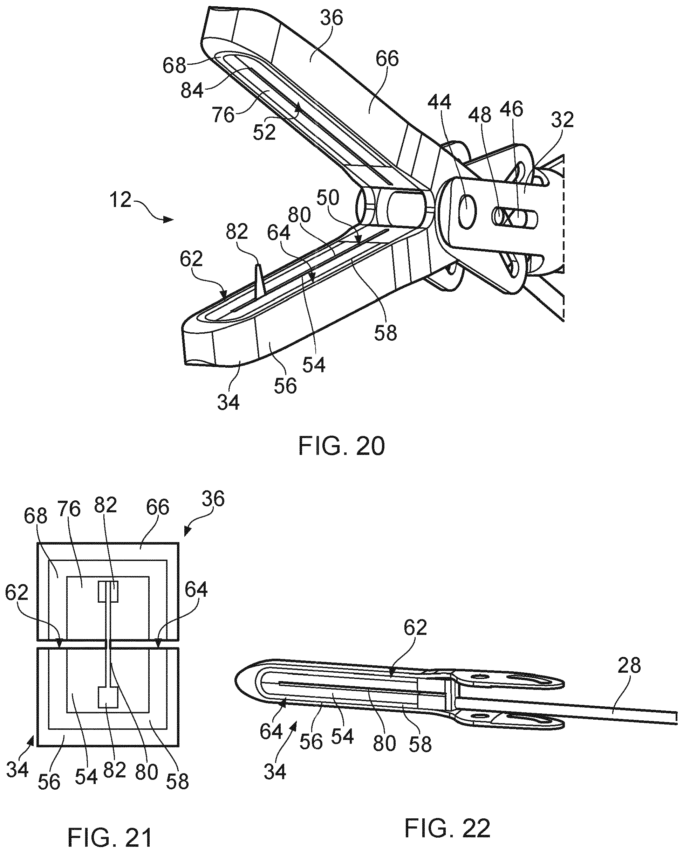

The embodiment of the electrosurgical instrument 12 shown in Figs. 20 to 22 includes the same features, characteristics, and/or optional embodiment as the embodiments of the electrosurgical instrument 12 shown in Fig. 18 except for the following differences.

The upper jaw 36 includes the third electrode 76 and the fourth electrode 66 which are separated by the fourth isolating portion 68. The third electrode 76 is electrically connected to the first electrode 54 and the fourth electrode 66 is electrically connected to the second electrode 56. The third electrode 76 and fourth electrode 66 are configured to emit microwave energy. No second isolating portion 60 and no third isolating portion 74 is provided. The first electrode 54 and the second electrode 56 may be mirror-symmetrically arranged with respect to the third electrode 76 and the fourth electrode 66.

The first groove 80 and second groove 84 are provided in the first electrode 54 and the third electrode 76, respectively. Thus, the blade 82 can be in electrical contact with the first electrode 54 and/or the third electrode 76. In this embodiment, the blade 82 is not configured to emit radiofrequency energy so that the blade 82 may be considered providing a "cold cut", i.e. only a mechanical cut.

The first sealing area 62 and the second sealing area 64 corresponds to the exposed sections of the first electrode 54 and the second electrode 56 that are close to the exposed sections of the first isolating portion 58. This is due to the fact that the intensity of the emitted microwave energy is highest between the exposed sections of the first electrode 54 and the second electrode 56 as demonstrated by the above-described simulations. Thus, here again, the first sealing area 62 and the second sealing area 64 (the areas where tissue between the first jaw 34 and the second jaw 36 is sealed using microwave energy) are spatially separated from the cutting line defined by the groove 80 or the blade 82.

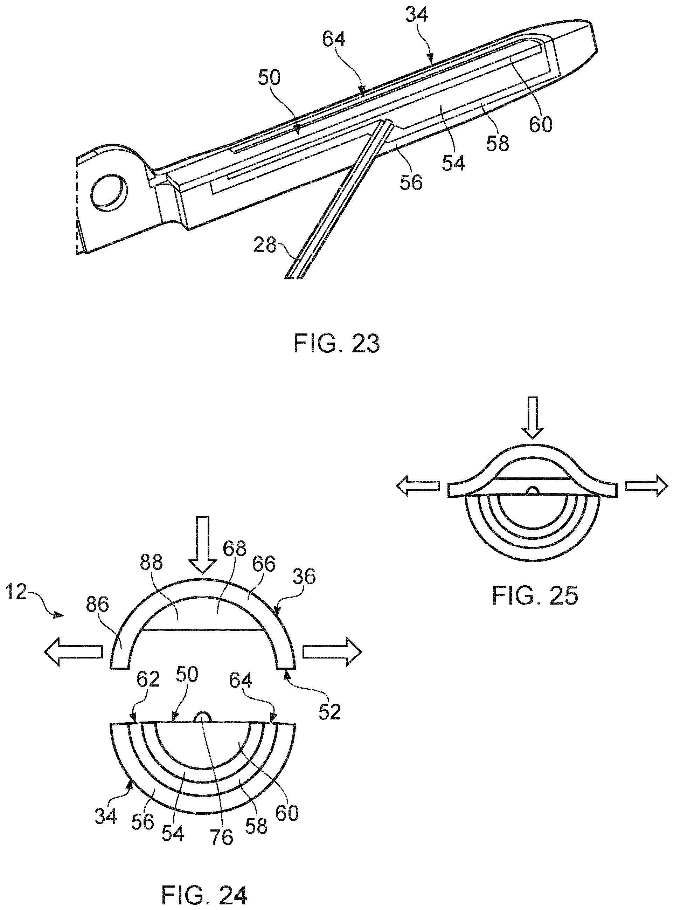

The embodiment of the electrosurgical instrument 12 shown in Fig. 23 includes the same features, characteristics, and/or optional embodiment as the embodiments of the electrosurgical instrument 12 shown in Fig. 10 except for the following differences.

The coaxial transmission line 28 is connected to the first electrode 54 and the second electrode 56 approximately in the middle of the first jaw 34. This is in contrast to the embodiments described above where the transmission line 28 is connected to the first electrode 54 and the second electrode 56 at a proximal end of the first jaw 34. So the first jaw 34 of Fig. 23 has a different point of connection compared to the first jaw 34 of Fig. 10. The emission distribution of microwave energy depends on the point where the transmission line 28 is connected to the first electrode 54 and the second electrode 56. Thus, the emission distribution can be changed by varying the point of connection.

This can be used for smoothing the distribution of microwave energy. For example, the jaw shown in Fig. 23 can be used as the second jaw 36 and jaw shown in Fig. 10 can be used as a first jaw 34. Since the energy distribution of the jaw of Fig. 23 is different from the energy distribution of the jaw of Fig. 10, the combined energy distributions may be smoother compared to a situation where the first jaw 34 and the second jaw 36 have the configuration as shown in Fig. 10.

The embodiment of the electrosurgical instrument 12 shown in Fig. 24 includes the same features, characteristics, and/or optional embodiment as the embodiments of the electrosurgical instrument 12 shown in Fig. 10 except for the following differences.

The second jaw 36 includes a protruding portion 86 and a recessed portion 88. The protruding portion 86 can be made from a flexible material, such as a plastic material. The protruding portion 86 can be covered by an electrically conductive layer for providing the fourth electrode 66. Distal end faces of the protruding portion 86 define the second surface 52.

The recessed portion 88 may be provided by the fourth isolating portion 68. In the closed position, the recessed portion 88 does not contact the third electrode 76, i.e. there is a gap between third electrode 76 and the recessed portion 88. Thus, in the closed position, little or no pressure is applied to the tissue between the third electrode 76 and the recessed portion 88 compared to the pressure applied to the tissue between the first sealing area 62 and the second sealing area 64 and the second surface 52 where the microwave sealing takes place.

As illustrated in Fig. 25, due to the flexibility of the protruding portion 86, the second jaw 36 can be further moved towards the first jaw 34 so that pressure can be applied on the tissue between the third electrode 76 and the recessed portion 88 for effecting radiofrequency cutting. The protruding portion 86 may bend outwards when the second jaw 36 is moved towards the first jaw 34 beyond the closed position as indicated by the arrows in Figs. 24 and 25.

## Claims

1. An electrosurgical instrument (12) for sealing and cutting tissue, comprising
an instrument shaft (30) comprising a transmission line (28) for conveying microwave electromagnetic energy,
a first jaw (34) attached to the instrument shaft (30) and including a first surface (50),
a second jaw (36) attached to the instrument shaft (30) and including a second surface (52),
a first electrode (54) for emitting microwave electromagnetic energy,
a second electrode (56) for emitting microwave electromagnetic energy,
a first isolating portion (58) electrically isolating the first electrode (54) from the second electrode (56),
a second isolating portion (60), and
a cutting device for cutting the tissue, and
wherein the first jaw (34) and the second jaw (36) can be moved between an open position, in which the tissue can be inserted between the first surface (50) and the second surface (52), and a closed position, in which the first and second surfaces (50, 52) are brought together to clamp tissue therebetween,
wherein the first electrode (54) and the second electrode (56) are arranged on the first jaw (34),
wherein sections of the first and second electrodes (54, 56) are exposed on the first surface (50) to define a first sealing area (62) and a second sealing area (64) on the first surface (50),
wherein the first sealing area (62) is spaced from the second sealing area (64) to form a gap therebetween on the first surface (50) so that the tissue between the first sealing area (62) and the second sealing area (64) is not sealed upon the emission of the microwave electromagnetic energy,
wherein the first and second sealing areas (62, 64) are configured to seal the tissue on either side of the gap using the emitted microwave electromagnetic energy, and
wherein the cutting device is effective along a cutting line on the first surface (50) to cut the tissue in the closed position, the cutting line being positioned on the gap,
**characterized in that**
the cutting device includes a radiofrequency electrode arranged on the cutting line,
wherein the second isolating portion (60) electrically isolates the radiofrequency electrode from the first electrode.

2. The electrosurgical instrument of claim 1, wherein the second electrode (56) covers the first electrode (54) on the side of the first electrode (54) facing away from the first surface (50) and/or wherein the first isolating portion (58) is arranged between the first electrode (54) and the second electrode (56).

3. The electrosurgical instrument of claim 1 or 2, wherein portions of the first electrode (54) and/or the second electrode (56) are plate-shaped and respectively include end faces, wherein the end faces form the exposed sections, wherein, in a cross-sectional view of the first jaw (34), the portions of the first electrode (54) and/or the second electrode (56) are substantially U-shaped or V-shaped.

4. The electrosurgical instrument of any preceding claim, wherein the exposed sections of the first electrode (54) and the second electrode (56):
at least partially extend parallel to each other,
each include two straight portions that extend parallel to each other, and/or
each form a loop, optionally a U-shape in a top view of the first jaw (34),
wherein optionally, in the closed position, the cutting line is arranged:
between the straight portions of the exposed sections of the first electrode (54) and/or
within the loop of the exposed section of the first electrode (54).

5. The electrosurgical instrument of any preceding claim, wherein the first sealing area (62) and the second sealing area (64) define an angle of less than 180° with respect to each other.

6. The electrosurgical instrument of any preceding claim, wherein the radiofrequency electrode includes a ridge protruding from the second isolating portion.

7. An electrosurgical instrument for sealing and cutting tissue, comprising
an instrument shaft (30) comprising a transmission line (28) for conveying microwave electromagnetic energy;
a first jaw (34) attached to the instrument shaft (30) and including a first surface (50),
a second jaw (36) attached to the instrument shaft (30) and including a second surface (52),
a first electrode (54) for emitting microwave electromagnetic energy,
a second electrode (56) for emitting microwave electromagnetic energy,
a first isolating portion (58) electrically isolating the first electrode (54) from the second electrode (56), and
a cutting device for cutting the tissue, and
wherein the first jaw (34) and the second jaw (36) can be moved between an open position, in which the tissue can be inserted between the first surface (50) and the second surface (52), and a closed position, in which the first and second surfaces (50, 52) are brought together to clamp tissue therebetween,
wherein the first electrode (54) and the second electrode (56) are arranged on the first jaw (34),
wherein sections of the first and second electrodes (54, 56) are exposed on the first surface (50) to define a first sealing area (62) and a second sealing area (64) on the first surface (50),
wherein the first sealing area (62) is spaced from the second sealing area (64) to form a gap therebetween on the first surface (50) so that the tissue between the first sealing area (62) and the second sealing area (64) is not sealed upon the emission of the microwave electromagnetic energy,
wherein the first and second sealing areas (62, 64) are configured to seal the tissue on either side of the gap using the emitted microwave electromagnetic energy, and
wherein the cutting device is effective along a cutting line on the first surface (50) to cut the tissue in the closed position, the cutting line being positioned on the gap,
**characterized in that**
the cutting device includes a radiofrequency electrode exposed on the second surface (52),
wherein the electrosurgical instrument further comprises a second isolating portion (60) arranged on the first jaw (34) so that, in the closed position, the second isolating portion (60) electrically isolates the radiofrequency electrode from the first electrode (54).

8. The electrosurgical instrument of claim 7,
wherein the second isolating portion (60) is made from silicon.

9. The electrosurgical instrument of any preceding claim, wherein the second jaw (36) includes a first arm (70) movable with respect to the second surface (52), wherein the second jaw (36) includes an opening through which the first arm (70) can be moved,
wherein optionally the radiofrequency electrode is arranged on the first arm (70),
wherein optionally the first arm (70) includes a third isolating portion (74) which is in contact with the radiofrequency electrode in a press position of the first arm (70), in which the first arm (70) and the first surface (50) are brought together to cut tissue therebetween,
wherein further optionally the second jaw (36) includes a fourth isolating portion (68) which covers the opening and is flexible across the opening so that the radiofrequency electrode is configured to deform the fourth isolating portion (68) in the closed position or the first arm (70) deforms the fourth isolating portion (68) in a press position of the first arm (70), in which the first arm (70) and the first surface (50) are brought together to cut tissue therebetween, wherein optionally the fourth isolating portion (68) is made from silicon.

10. The electrosurgical instrument of claim 6, wherein the second jaw (36) includes a protruding portion (86) and a recessed portion (88), wherein, in the closed position, the protruding portion (86) is in contact with the first surface (50) and the recessed portion (88) is spaced from the radiofrequency electrode, and wherein the protruding portion (86) is flexible so that the second jaw (36) can be further moved towards the first surface (50) relative to the closed position so that the recessed portion (88) can be moved closer towards the radiofrequency electrode.

11. The electrosurgical instrument of any preceding claim, further comprising a third electrode (76) for emitting microwave energy and exposed on the second surface (52) and/or a fourth electrode (66) for emitting microwave energy and exposed on the second surface (52),
wherein optionally
the first electrode (54) is electrically connected to the third electrode (76) and/or the second electrode (56) is electrically connected to the fourth electrode (66), and/or
the transmission line (28) comprises a first coaxial cable (38), which is connected to the first electrode (54) and the second electrode (56), and a second coaxial cable (40), which is connected to the third electrode (76) and the fourth electrode (66), wherein optionally, in the closed position, a first point of contact of the first coaxial cable (38) with the first electrode (54) and the second electrode (56) is laterally offset along a direction of extension of the first jaw (34) and the second jaw (36) compared to a second point of contact of the second coaxial cable (40) with the third electrode (76) and the fourth electrode (66).

12. An electrosurgical apparatus for sealing and cutting tissue, comprising
a generator unit (14) for generating radiofrequency and/or microwave electromagnetic energy, and
the electrosurgical instrument according to any preceding claim,
wherein the transmission line (28) conveys the radiofrequency and/or microwave electromagnetic energy from the generator unit (14) to the first electrode (54), the second electrode (56) and/or the cutting device.

13. The electrosurgical apparatus of claim 12, wherein the generator unit (14) is configured to simultaneously generate microwave electromagnetic energy of a first frequency and microwave electromagnetic energy of a second frequency.

14. The electrosurgical apparatus of claim 13, wherein the first coaxial cable (38) is connected to the generator unit (14) to receive the first frequency and the second coaxial cable (40) is connected to the generator unit (14) to receive the second frequency.

15. The electrosurgical apparatus of any one of the claims 12 to 14, wherein the generator unit (14) is further configured to simultaneously or alternatingly generate microwave electromagnetic energy of the first frequency and radiofrequency electromagnetic energy of a third frequency.

## Patentansprüche

1. Elektrochirurgisches Instrument (12) zum Versiegeln und Schneiden von Gewebe, das Folgendes umfasst:
einen Instrumentenschaft (30), der eine Übertragungsleitung (28) zum Übertragen von elektromagnetischer Mikrowellenenergie umfasst,
eine erste Backe (34), die am Instrumentenschaft (30) befestigt ist und eine erste Oberfläche (50) umfasst,
eine zweite Backe (36), die am Instrumentenschaft (30) befestigt ist und eine zweite Oberfläche (52) umfasst,
eine erste Elektrode (54) zum Emittieren von elektromagnetischer Mikrowellenenergie
eine zweite Elektrode (56) zum Emittieren von elektromagnetischer Mikrowellenenergie,
einen ersten Isolierabschnitt (58), der die erste Elektrode (54) elektrisch von der zweiten Elektrode (56) isoliert,
einen zweiten Isolierabschnitt (60) und
eine Schneidevorrichtung zum Schneiden des Gewebes, und
wobei die erste Backe (34) und die zweite Backe (36) zwischen einer offenen Stellung, in der das Gewebe zwischen die erste Oberfläche (50) und die zweite Oberfläche (52) eingeführt werden kann, und einer geschlossenen Stellung, in der die erste und zweite Oberfläche (50, 52) zusammengebracht sind, um das Gewebe zwischen diesen einzuklemmen, bewegt werden können,
wobei die erste Elektrode (54) und die zweite Elektrode (56) auf der ersten Backe (34) angeordnet sind,
wobei Abschnitte der ersten und zweiten Elektrode (54, 56) auf der ersten Oberfläche (50) exponiert sind, um einen ersten Versiegelungsbereich (62) und einen zweiten Versiegelungsbereich (64) auf der ersten Oberfläche (50) zu definieren,
wobei der erste Versiegelungsbereich (62) vom zweiten Versiegelungsbereich (64) beabstandet ist, um einen Spalt zwischen diesen auf der ersten Oberfläche (50) zu bilden, sodass das Gewebe zwischen dem ersten Versiegelungsbereich (62) und dem zweiten Versiegelungsbereich (64) bei der Emission der elektromagnetischen Mikrowellenenergie nicht versiegelt wird,
wobei der erste und zweite Versiegelungsbereich (62, 64) ausgelegt sind, das Gewebe auf beiden Seiten des Spalts unter Verwendung der emittierten elektromagnetischen Mikrowellenenergie zu versiegeln, und
wobei die Schneidevorrichtung entlang einer Schneidelinie auf der ersten Oberfläche (50) wirksam ist, um das Gewebe in der geschlossenen Stellung zu schneiden, wobei die Schneidelinie auf dem Spalt positioniert ist,
**dadurch gekennzeichnet, dass**
die Schneidevorrichtung eine Hochfrequenzelektrode umfasst, die auf der Schneidelinie angeordnet ist,
wobei der zweite Isolierabschnitt (60) die Hochfrequenzelektrode elektrisch von der ersten Elektrode isoliert.

2. Elektrochirurgisches Instrument nach Anspruch 1, wobei die zweite Elektrode (56) die erste Elektrode (54) auf der Seite der ersten Elektrode (54) abdeckt, die von der ersten Oberfläche (50) weg weist, und/oder wobei der erste Isolierabschnitt (58) zwischen der ersten Elektrode (54) und der zweiten Elektrode (56) angeordnet ist.

3. Elektrochirurgisches Instrument nach Anspruch 1 oder 2, wobei Abschnitte der ersten Elektrode (54) und/oder der zweiten Elektrode (56) plattenförmig sind und jeweils Endflächen umfassen, wobei die Endflächen die exponierten Abschnitte bilden, wobei die Abschnitte der ersten Elektrode (54) und/oder der zweiten Elektrode (56) in einer Querschnittsansicht der ersten Backe (34) im Wesentlichen U-förmig oder V-förmig sind.

4. Elektrochirurgisches Instrument nach einem der vorangegangenen Ansprüche, wobei die exponierten Abschnitte der ersten Elektrode (54) und der zweiten Elektrode (56):
sich zumindest teilweise parallel zueinander erstrecken,
jeweils zwei gerade Abschnitte umfassen, die sich parallel zueinander erstrecken, und/oder
jeweils eine Schleife bilden, die gegebenenfalls in einer Draufsicht der ersten Backe (34) U-förmig ist,
wobei die Schneidelinie in der geschlossenen Stellung gegebenenfalls wie folgt angeordnet ist:
zwischen den geraden Abschnitten der exponierten Abschnitte der ersten Elektrode (54) und/oder
innerhalb der Schleife des exponierten Abschnitts der ersten Elektrode (54).

5. Elektrochirurgisches Instrument nach einem der vorangegangenen Ansprüche, wobei der erste Versiegelungsbereich (62) und der zweite Versiegelungsbereich (64) einen Winkel von weniger als 180° in Bezug zueinander definieren.

6. Elektrochirurgisches Instrument nach einem der vorangegangenen Ansprüche, wobei die Hochfrequenzelektrode einen Steg umfasst, der vom zweiten Isolierabschnitt vorsteht.

7. Elektrochirurgisches Instrument zum Versiegeln und Schneiden von Gewebe, das Folgendes umfasst:
einen Instrumentenschaft (30), der eine Übertragungsleitung (28) zum Übertragen von elektromagnetischer Mikrowellenenergie umfasst,
eine erste Backe (34), die am Instrumentenschaft (30) befestigt ist und eine erste Oberfläche (50) umfasst,
eine zweite Backe (36), die am Instrumentenschaft (30) befestigt ist und eine zweite Oberfläche (52) umfasst,
eine erste Elektrode (54) zum Emittieren von elektromagnetischer Mikrowellenenergie
eine zweite Elektrode (56) zum Emittieren von elektromagnetischer Mikrowellenenergie,
einen ersten Isolierabschnitt (58), der die erste Elektrode (54) elektrisch von der zweiten Elektrode (56) isoliert,
eine Schneidevorrichtung zum Schneiden des Gewebes, und
wobei die erste Backe (34) und die zweite Backe (36) zwischen einer offenen Stellung, in der das Gewebe zwischen die erste Oberfläche (50) und die zweite Oberfläche (52) eingeführt werden kann, und einer geschlossenen Stellung, in der die erste und zweite Oberfläche (50, 52) zusammengebracht sind, um das Gewebe zwischen diesen einzuklemmen, bewegt werden können,
wobei die erste Elektrode (54) und die zweite Elektrode (56) auf der ersten Backe (34) angeordnet sind,
wobei Abschnitte der ersten und zweiten Elektrode (54, 56) auf der ersten Oberfläche (50) exponiert sind, um einen ersten Versiegelungsbereich (62) und einen zweiten Versiegelungsbereich (64) auf der ersten Oberfläche (50) zu definieren,
wobei der erste Versiegelungbereich (62) vom zweiten Versiegelungsbereich (64) beabstandet ist, um einen Spalt zwischen diesen auf der ersten Oberfläche (50) zu bilden, sodass das Gewebe zwischen dem ersten Versiegelungsbereich (62) und dem zweiten Versiegelungsbereich (64) bei der Emission der elektromagnetischen Mikrowellenenergie nicht versiegelt wird,
wobei der erste und zweite Versiegelungsbereich (62, 64) ausgelegt sind, das Gewebe auf beiden Seiten des Spalts unter Verwendung der emittierten elektromagnetischen Mikrowellenenergie zu versiegeln, und
wobei die Schneidevorrichtung entlang einer Schneidelinie auf der ersten Oberfläche (50) wirksam ist, um das Gewebe in der geschlossenen Stellung zu schneiden, wobei die Schneidelinie auf dem Spalt positioniert ist,
**dadurch gekennzeichnet, dass**
die Schneidevorrichtung eine Hochfrequenzelektrode umfasst, die auf der zweiten Oberfläche (52) exponiert ist,
wobei das elektrochirurgische Instrument weiters einen zweiten Isolierabschnitt (60) umfasst, der auf der ersten Backe (34) angeordnet ist, sodass der zweite Isolierabschnitt (60) in der geschlossenen Stellung die Hochfrequenzelektrode elektrisch von der ersten Elektrode (54) isoliert.

8. Elektrochirurgisches Instrument nach Anspruch 7, wobei der zweite Isolierabschnitt (60) aus Silicium besteht.

9. Elektrochirurgisches Instrument nach einem der vorangegangenen Ansprüche, wobei die zweite Backe (36) einen ersten Arm (70) umfasst, der in Bezug auf die zweite Oberfläche (52) bewegbar ist, wobei die zweite Backe (36) eine Öffnung umfasst, durch die der erste Arm (70) bewegt werden kann,
wobei die Hochfrequenzelektrode gegebenenfalls am ersten Arm (70) angeordnet ist,
wobei der erste Arm (70) gegebenenfalls einen dritten Isolierabschnitt (74) umfasst, der in einer Pressstellung des ersten Arms (70), in welcher der erste Arm (70) und die erste Oberfläche (50) zusammengebracht sind, um Gewebe zwischen diesen zu schneiden, mit der Hochfrequenzelektrode in Kontakt ist,
wobei die zweite Backe (36) gegebenenfalls einen vierten Isolierabschnitt (68) umfasst, der die Öffnung abdeckt und über der Öffnung flexibel ist, sodass die Hochfrequenzelektrode ausgelegt ist, den vierten Isolierabschnitt (68) in der geschlossenen Stellung zu verformen, oder der erste Arm (70) verformt den vierten Isolierabschnitt (68) in einer Pressstellung des ersten Arms (70), in welcher der erste Arm (70) und die erste Oberfläche (50) zusammengebracht sind, um Gewebe zwischen diesen zu schneiden, wobei der vierte Isolierabschnitt (68) gegebenenfalls aus Silicium besteht.

10. Elektrochirurgisches Instrument nach Anspruch 6, wobei die zweite Backe (36) einen vorstehenden Abschnitt (86) und einen vertieften Abschnitt (88) umfasst, wobei in der geschlossenen Stellung der vorstehende Abschnitt (86) mit der ersten Oberfläche (50) in Kontakt ist und der vertiefte Abschnitt (88) von der Hochfrequenzelektrode beabstandet ist, und wobei der vorstehende Abschnitt (86) flexibel ist, sodass die zweite Backe (36) relativ zur geschlossenen Stellung weiter zur ersten Oberfläche (50) hin bewegt werden kann, sodass der vertiefte Abschnitt (88) näher zur Hochfrequenzelektrode hin bewegt werden kann.

11. Elektrochirurgisches Instrument nach einem der vorangegangenen Ansprüche, das weiters eine dritte Elektrode (76) zum Emittieren von Mikrowellenenergie, die auf der zweiten Oberfläche (52) exponiert ist, und/oder eine vierte Elektrode (66) zum Emittieren von Mikrowellenergie, die auf der zweiten Oberfläche (52) exponiert ist, umfasst, wobei gegebenenfalls
die erste Elektrode (54) elektrisch mit der dritten Elektrode (76) verbunden ist und/oder die zweite Elektrode (56) elektrisch mit der vierten Elektrode (66) verbunden ist, und/oder
die Übertragungsleitung (28) ein erstes Koaxialkabel (38), das mit der ersten Elektrode (54) und der zweiten Elektrode (56) verbunden ist, und ein zweites Koaxialkabel (40) umfasst, das mit der dritten Elektrode (76) und der vierten Elektrode (66) verbunden ist, wobei gegebenenfalls in der geschlossenen Stellung ein erster Kontaktpunkt des ersten Koaxialkabels (38) mit der ersten Elektrode (54) und der zweiten Elektrode (56) im Vergleich zu einem zweiten Kontaktpunkt des zweiten Koaxialkabels (40) mit der dritten Elektrode (76) und der vierten Elektrode (66) entlang der Ausdehnungsrichtung der ersten Backe (34) und der zweiten Backe (36) seitlich versetzt ist.

12. Elektrochirurgisches Gerät zum Versiegeln und Schneiden von Gewebe, das Folgendes umfasst:
eine Generatoreinheit (14) zum Erzeugen von Hochfrequenz- und/oder elektromagnetischer Mikrowellenenergie und
ein elektrochirurgisches Instrument nach einem der vorangegangenen Ansprüche,
wobei die Übertragungsleitung (28) die Hochfrequenz- und/oder elektromagnetische Mikrowellenenergie von der Generatoreinheit (14) zur ersten Elektrode (54), zur zweiten Elektrode (56) und/oder zur Schneidevorrichtung überträgt.

13. Elektrochirurgisches Gerät nach Anspruch 12, wobei die Generatoreinheit (14) ausgelegt ist, gleichzeitig elektromagnetische Mikrowellenenergie mit einer ersten Frequenz und elektromagnetische Mikrowellenenergie mit einer zweiten Frequenz zu erzeugen.

14. Elektrochirurgisches Gerät nach Anspruch 13, wobei das erste Koaxialkabel (38) mit der Generatoreinheit (14) verbunden ist, um die erste Frequenz zu empfangen, und das zweite Koaxialkabel (40) mit der Generatoreinheit (14) verbunden ist, um die zweite Frequenz zu empfangen.

15. Elektrochirurgisches Gerät nach einem der Ansprüche 12 bis 14, wobei die Generatoreinheit (14) weiters ausgelegt ist, gleichzeitig oder abwechselnd elektromagnetische Mikrowellenenergie mit der ersten Frequenz und elektromagnetische Hochfrequenzenergie mit einer dritten Frequenz zu erzeugen.

## Revendications

1. Instrument électrochirurgical (12) pour sceller et couper un tissu, comprenant
un tige d'instrument (30) comprenant une ligne de transmission (28) pour acheminer de l'énergie électromagnétique hyperfréquence ;
une première mâchoire (34) fixée à la tige d'instrument (30) et incluant une première surface (50),
une seconde mâchoire (36) fixée à la tige d'instrument (30) et incluant une seconde surface (52),
une première électrode (54) pour émettre une énergie électromagnétique hyperfréquence,
une deuxième électrode (56) pour émettre une énergie électromagnétique hyperfréquence,
une première partie d'isolation (58) isolant électriquement la première électrode (54) de la deuxième électrode (56),
une seconde partie d'isolation (60), et
un dispositif de coupe pour couper le tissu, et
dans lequel la première mâchoire (34) et la seconde mâchoire (36) peuvent être déplacées entre une position ouverte, dans laquelle le tissu peut être inséré entre la première surface (50) et la seconde surface (52), et une position fermée, dans laquelle les première et seconde surfaces (50, 52) sont amenées ensemble pour serrer un tissu entre celles-ci,
dans lequel la première électrode (54) et la deuxième électrode (56) sont agencées sur la première mâchoire (34),
dans lequel des sections des première et deuxième électrodes (54, 56) sont exposées sur la première surface (50) pour définir une première zone d'étanchéité (62) et une seconde zone d'étanchéité (64) sur la première surface (50),
dans lequel la première zone d'étanchéité (62) est espacée de la seconde zone d'étanchéité (64) pour former un espace entre celles-ci sur la première surface (50) de telle sorte que le tissu entre la première zone d'étanchéité (62) et la seconde zone d'étanchéité (64) n'est pas scellé lors de l'émission d'énergie électromagnétique hyperfréquence,
dans lequel les première et seconde zones d'étanchéité (62, 64) sont configurées pour sceller le tissu de chaque côté de l'espace en utilisant l'énergie électromagnétique hyperfréquence émise, et
dans lequel le dispositif de coupe est efficace le long d'une ligne de coupe sur la première surface (50) pour couper le tissu dans la position fermée, la ligne de coupe étant positionnée sur l'espace,
**caractérisé en ce que**
le dispositif de coupe inclut une électrode radiofréquence agencée sur la ligne de coupe,
dans lequel la deuxième partie d'isolation (60) isole électriquement l'électrode radiofréquence de la première électrode.

2. Instrument électrochirurgical selon la revendication 1, dans lequel la deuxième électrode (56) recouvre la première électrode (54) sur le côté de la première électrode (54) orienté à l'opposé de la première surface (50) et/ou dans lequel la première partie d'isolation (58) est agencée entre la première électrode (54) et la deuxième électrode (56).

3. Instrument électrochirurgical selon la revendication 1 ou 2, dans lequel des parties de la première électrode (54) et/ou de la deuxième électrode (56) sont en forme de plaque et incluent respectivement des faces d'extrémité, dans lequel les faces d'extrémité forment les sections exposées, dans lequel, dans une vue en coupe transversale de la première mâchoire (34), les parties de la première électrode (54) et/ou de la deuxième électrode (56) sont sensiblement en forme de U ou en forme de V.

4. Instrument électrochirurgical selon l'une quelconque des revendications précédentes, dans lequel les sections exposées de la première électrode (54) et de la deuxième électrode (56) :
s'étendent au moins partiellement parallèlement les unes aux autres,
Incluent chacune deux parties droites qui s'étendent parallèlement l'une à l'autre, et/ou
forment chacune une boucle, facultativement une forme en U dans une vue de dessus de la première mâchoire (34),
dans lequel facultativement, dans la position fermée, la ligne de coupe est agencée :
entre les parties droites des sections exposées de la première électrode (54) et/ou
dans la boucle de la section exposée de la première électrode (54).

5. Instrument électrochirurgical selon l'une quelconque des revendications précédentes, dans lequel la première zone d'étanchéité (62) et la seconde zone d'étanchéité (64) définissent un angle inférieur à 180° l'une par rapport à l'autre.

6. Instrument électrochirurgical selon l'une quelconque des revendications précédentes, dans lequel l'électrode radiofréquence inclut une arête faisant saillie à partir de la deuxième partie d'isolation.

7. Instrument électrochirurgical pour sceller et couper un tissu, comprenant
un tige d'instrument (30) comprenant une ligne de transmission (28) pour acheminer de l'énergie électromagnétique hyperfréquence ;
une première mâchoire (34) fixée à la tige d'instrument (30) et incluant une première surface (50),
une seconde mâchoire (36) fixée à la tige d'instrument (30) et incluant une seconde surface (52),
une première électrode (54) pour émettre une énergie électromagnétique hyperfréquence,
une deuxième électrode (56) pour émettre une énergie électromagnétique hyperfréquence,
une première partie d'isolation (58) isolant électriquement la première électrode (54) de la deuxième électrode (56), et
un dispositif de coupe pour couper le tissu, et
dans lequel la première mâchoire (34) et la seconde mâchoire (36) peuvent être déplacées entre une position ouverte, dans laquelle le tissu peut être inséré entre la première surface (50) et la seconde surface (52), et une position fermée, dans laquelle les première et seconde surfaces (50, 52) sont amenées ensemble pour serrer un tissu entre celles-ci,
dans lequel la première électrode (54) et la deuxième électrode (56) sont agencées sur la première mâchoire (34),
dans lequel des sections des première et deuxième électrodes (54, 56) sont exposées sur la première surface (50) pour définir une première zone d'étanchéité (62) et une seconde zone d'étanchéité (64) sur la première surface (50),
dans lequel la première zone d'étanchéité (62) est espacée de la seconde zone d'étanchéité (64) pour former un espace entre celles-ci sur la première surface (50) de telle sorte que le tissu entre la première zone d'étanchéité (62) et la seconde zone d'étanchéité (64) n'est pas scellé lors de l'émission d'énergie électromagnétique hyperfréquence,
dans lequel les première et seconde zones d'étanchéité (62, 64) sont configurées pour sceller le tissu de chaque côté de l'espace en utilisant l'énergie électromagnétique hyperfréquence émise, et
dans lequel le dispositif de coupe est efficace le long d'une ligne de coupe sur la première surface (50) pour couper le tissu dans la position fermée, la ligne de coupe étant positionnée sur l'espace,
**caractérisé en ce que**
le dispositif de coupe inclut une électrode radiofréquence exposée sur la seconde surface (52),
dans lequel l'instrument électrochirurgical comprend en outre une deuxième partie d'isolation (60) agencée sur la première mâchoire (34) de sorte que dans la position fermée, la deuxième partie d'isolation (60) isole électriquement l'électrode radiofréquence de la première électrode (54).

8. Instrument électrochirurgical selon la revendication 7, dans lequel la deuxième partie d'isolation (60) est réalisée en silicium.

9. Instrument électrochirurgical selon l'une quelconque des revendications précédentes, dans lequel la seconde mâchoire (36) inclut un premier bras (70) mobile par rapport à la seconde surface (52), dans lequel la seconde mâchoire (36) inclut une ouverture à travers laquelle le premier bras (70) peut être déplacé,
dans lequel facultativement l'électrode radiofréquence est agencée sur le premier bras (70),
dans lequel facultativement le premier bras (70) inclut une troisième partie d'isolation (74) qui est en contact avec l'électrode radiofréquence dans une position de pression du premier bras (70), dans laquelle le premier bras (70) et la première surface (50) sont rapprochés pour couper du tissu entre eux,
dans lequel en outre facultativement la seconde mâchoire (36) inclut une quatrième partie d'isolation (68) qui recouvre l'ouverture et est flexible à travers l'ouverture de telle sorte que l'électrode radiofréquence est configurée pour déformer la quatrième partie d'isolation (68) dans la position fermée ou le premier bras (70) déforme la quatrième partie d'isolation (68) dans une position de pression du premier bras (70), dans laquelle le premier bras (70) et la première surface (50) sont rapprochés pour couper du tissu entre eux, dans lequel facultativement la quatrième partie d'isolation (68) est réalisée en silicium.

10. Instrument électrochirurgical selon la revendication 6, dans lequel la seconde mâchoire (36) inclut une partie en saillie (86) et une partie en retrait (88), dans lequel, dans la position fermée, la partie en saillie (86) est en contact avec la première surface (50) et la partie en retrait (88) est espacée de l'électrode radiofréquence, et dans lequel la partie en saillie (86) est flexible de telle sorte que la seconde mâchoire (36) puisse être déplacée davantage vers la première surface (50) par rapport à la position fermée de telle sorte que la partie en retrait (88) puisse être rapprochée de l'électrode radiofréquence.

11. Instrument électrochirurgical selon l'une quelconque des revendications précédentes, comprenant en outre une troisième électrode (76) pour émettre une énergie hyperfréquence et exposée sur la seconde surface (52) et/ou une quatrième électrode (66) pour émettre une énergie hyperfréquence et exposée sur la seconde surface (52),
dans lequel facultativement
la première électrode (54) est connectée électriquement à la troisième électrode (76) et/ou la deuxième électrode (56) est connectée électriquement à la quatrième électrode (66), et/ou
la ligne de transmission (28) comprend un premier câble coaxial (38), qui est connecté à la première électrode (54) et à la deuxième électrode (56), et un second câble coaxial (40), qui est connecté à la troisième électrode (76) et à la quatrième électrode (66), dans lequel facultativement, dans la position fermée, un premier point de contact du premier câble coaxial (38) avec la première électrode (54) et la deuxième électrode (56) est décalé latéralement le long d'une direction d'extension de la première mâchoire (34) et de la seconde mâchoire (36) par comparaison à un second point de contact du second câble coaxial (40) avec la troisième électrode (76) et la quatrième électrode (66).

12. Appareil électrochirurgical pour sceller et couper un tissu, comprenant
une unité de génération (14) pour générer de l'énergie électromagnétique radiofréquence et/ou hyperfréquence, et
un instrument électrochirurgical selon l'une quelconque des revendications précédentes,
dans lequel la ligne de transmission (28) achemine l'énergie électromagnétique radiofréquence et/ou hyperfréquence à partir de l'unité de générateur (14) vers la première électrode (54), la deuxième électrode (56) et/ou le dispositif de coupe.

13. Appareil électrochirurgical selon la revendication 12, dans lequel l'unité de générateur (14) est configurée pour générer simultanément une énergie électromagnétique hyperfréquence d'une première fréquence et une énergie électromagnétique hyperfréquence d'une deuxième fréquence.

14. Appareil électrochirurgical selon la revendication 13, dans lequel le premier câble coaxial (38) est connecté à l'unité de générateur (14) pour recevoir la première fréquence et le second câble coaxial (40) est connecté à l'unité de générateur (14) pour recevoir la deuxième fréquence.

15. Appareil électrochirurgical selon l'une quelconque des revendications 12 à 14, dans lequel l'unité de générateur (14) est en outre configurée pour générer simultanément ou alternativement une énergie électromagnétique hyperfréquence de la première fréquence et une énergie électromagnétique radiofréquence d'une troisième fréquence.
